# EUROPEAN PATENT SPECIFICATION

(11) **EP 1 030 836 B1**
(45) Date of publication and mention of the grant of the patent: **29.01.2003**
(21) Application number: 98952927.6
(22) Date of filing: 12.11.1998
(51) Int. Cl.: C07C 311/04, C07D 211/96, C07D 233/72, C07D 309/08, C07D 401/12, C07D 401/14, C07D 407/12, A61K 31/16, A61K 31/18, A61K 31/19, A61K 31/415

(54) **HYDROXAMIC AND CARBOXYLIC ACID DERIVATIVES HAVING MMP AND TNF INHIBITORY ACTIVITY**
HYDROXAMSÄURE- UND CARBONSÄUREDERIVATE MIT MMP UND TNF HEMMENDER WIRKUNG
DERIVES D'ACIDE HYDROXAMIQUE ET CARBOCYCLIQUE AYANT UNE ACTIVITE D'INHIBITION DES MMP ET DU TNF

(30) Priority: 12.11.1997 GB 9723906; 06.02.1998 GB 9802618; 26.06.1998 GB 9813933
(43) Date of publication of application: 30.08.2000
(73) Proprietor: Darwin Discovery Limited, Cambridge CB4 4WE (GB)
(72) Inventor: BAXTER, Andrew, Douglas, Darwin Discovery Limited, Cambridge CB4 4WE (GB); OWEN, David, Alan, Darwin Discovery Limited, Cambridge CB4 4WE (GB); MONTANA, John, Gary, Darwin Discovery Limited, Cambridge CB4 4WE (GB); WATSON, Robert, John, Darwin Discovery Limited, Cambridge CB4 4WE (GB)
(74) Representative: Perry, Robert Edward
(86) International application number: GB9803395
(87) International publication number: WO99024399

(56) References cited:
- GB-A- 1 067 965
- T. GRAAFLAND, ET AL.: "Structure and reactivity in tntramolecular catalysis. Catalysis of sulphonamide hydrolysis by the neighbouring carboxyl group" JOURNAL OF THE AMERICAN CHEMICAL SOCIETY, vol. 101, no. 23, 7 November 1979, pages 6981-6991, XP002070768 Washington, DC, US
- J. JAGER, ET AL.: "The Thorpe-Ingold effect in the intramolecular carboxyl-catalysed hydrolysis of sulphonamides" JOURNAL OF THE AMERICAN CHEMICAL SOCIETY, vol. 106, no. 1, 11 January 1984, pages 139-143, XP002070769 Washington, DC, US
- J.E. OLIVER, ET AL.: "A Knoevenagel-type synthesis of styrene-omega-sulphonanilides" SYNTHESIS, no. 5, May 1975, pages 321-322, XP002091870 Stuttgart, DE
- B.F. CAIN, ET AL.: "Potential antitumour agents. 23. 4'-(9-Acridinylamino)alkanesulphonamide congeners bearing hydrophilic functionality" JOURNAL OF MEDICINAL CHEMISTRY, vol. 20, no. 8, August 1977, pages 987-996, XP002091871 Washington, DC, US
- D.R. SHRIDHAR, ET AL.: "Synthesis and biological activity of some new 2-[(5-nitro-2-furyl- and 5-nitro- 2-thienyl)vinyl]-N-arylsulphonamides and 1-[2-(5-nitro-2-furyl- and 5-nitro-2-thienyl)vinyl]sulphonyl heterocycles" INDIAN JOURNAL OF CHEMISTRY, vol. 20B, no. 3, March 1981, pages 234-237, XP002091872 New Delhi, IN
- D.R. SHRIDHAR, ET AL.: "Synthesis and biological activity of some new 2-(heteroaryl)vinylsulphonyl derivatives" JOURNAL OF THE INDIAN JOURNAL CHEMICAL SOCIETY, vol. 62, no. 7, July 1985, pages 537-540, XP002091873 Calcutta, IN
- B.J.R. NICOLAUS, ET AL.: "Auf das Zentralnervensystem wirkende Substanzen XXXV. Über neuartige schwefelhaltige Heterocyclen II: Synthese und Eigenschaften der 3-Alkoxy-4,4-dialkyl- 4H-1,2-thiazet-1,1-dioxide" HELVETICA CHIMICA ACTA, vol. 46, no. 2, 15 March 1963, pages 450-461, XP002070767 Basel, CH
- C.P. DECICCO, ET AL.: "Amide surrogates of matrix metalloproteinase inhibitors: urea and sulphonamide mimics" BIOORGANIC AND MEDICINAL CHEMISTRY LETTERS, vol. 7, no. 18, 1997, pages 2331-2336, XP002071089 Oxford, GB

## Description

This invention relates to hydroxamic and carboxylic acid derivatives, and to their use in medicine.

### Background to the Invention

Metalloproteinases, including matrix metalloproteinases (MMPs), (human fibroblast) collagenase, gelatinase and TNF convertase (TACE), and their modes of action, and also inhibitors thereofand their clinical effects, are described in WO-A-9611209, WO-A-9712902 and WO-A-9719075, the contents of which are incorporated herein by reference. MMP inhibitors may also be useful in the inhibition of other mammalian metalloproteinases such as the adamalysin family (or ADAMs) whose members include TNF convertase (TACE) and ADAM-10, which can cause the release of TNFα from cells, and others, which have been demonstrated to be expressed by human articular cartilage cells and also involved in the destruction of myelin basic protein, a phenomenon associated with multiple sclerosis.

Compounds which have the property of inhibiting the action of metalloproteinases involved in connective tissue breakdown, such as collagenases, stromelysins and gelatinases, have been shown to inhibit the release of TNF both *in vitro* and *in vivo*. See Gearing *et al* (1994), Nature 370:555-557; McGeehan *et al* (1994), Nature 370:558-561; GB-A-2268934; and WO-A-9320047. All of these reported inhibitors contain a hydroxamic acid zinc-binding group, as do the imidazole-substituted compounds disclosed in WO-A-9523790. Other compounds that inhibit MMP and/or TNF are described in WO-A-9513289, WO-A-9611209, WO-A-96035687, WO-A-96035711, WO-A-96035712 and WO-A-96035714.

### Summary of the Invention

The invention encompasses compounds which are useful inhibitors of matrix metalloproteinases and/or TNFα-mediated diseases, including degenerative diseases and certain cancers. These compounds are represented by formula (I): 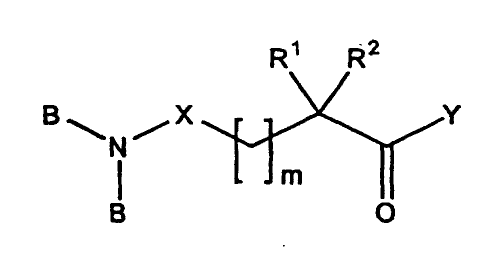 wherein
m is 0-2;
X is S(O)₁₋₂;
Y is OH or NHOH;
R¹ is H or a group (optionally substituted with R⁷) selected from C₁₋₆ alkyl, C₂₋₆ alkenyl, aryl, C₁₋₆ alkyl-aryl, heteroaryl, C₁₋₆ alkyl-heteroaryl, heterocycloalkyl, C₁₋₆ alkyl-heterocycloalkyl, cycloalkyl and C₁₋₆ alkyl-cycloalkyl, and R² is H or C₁₋₆ alkyl;
   or CR¹R² is a cycloalkyl or heterocycloalkyl ring optionally substituted with R⁷ or a group (optionally substituted with R⁷) selected from C₁₋₆ alkyl, aryl, C₁₋₆ alkyl-aryl, heteroaryl and C₁₋₆ alkyl-heteroaryl;
   each B is the same or different and is H or a group selected from C₁₋₆ alkyl-aryl, C₁₋₆ alkyl, cycloalkyl, C₁₋₆ alkyl-cycloalkyl, cycloalkenyl, heterocycloalkenyl, C₁₋₆ alkyl-heteroaryl, heterocycloalkyl, C₁₋₆ alkyl-heterocycloalkyl, aryl or heteroaryl, any of which groups is optionally substituted by a substituent selected from R³, C₁₋₆ alkyl-R³, C₂₋₆ alkenyl-R³, aryl (optionally substituted with R³), aryl-C₁₋₆ alkyl-R³, C₁₋₆ alkyl-aryl (optionally substituted with R³), C₁₋₆ alkyl-heteroaryl (optionally substituted with R³), aryl-C₂₋₆ alkenyl-R⁵, heteroaryl (optionally substituted with R³), heteroaryl-C₁₋₆, alkyl-R³, cycloalkyl (optionally substituted with R³) and heterocycloalkyl (optionally substituted with R³), provided that NB₂ is not NH₂,
   or B-N-B is a heterocycloalkyl ring substituted with =O or =NOR⁴,
   or, when neither of R¹ and R² is H, B-N-B is a heterocycloalkyl or heterocycloalkenyl ring optionally substituted by a substituent selected from R³, C₁₋₆ alkyl-R³, C₂₋₆ alkenyl-R³, aryl (optionally substituted with R³), aryl-C₁₋₆ alkyl-R³, C₁₋₆ alkyl-aryl (optionally substituted with R³), C₁₋₆ alkyl-heteroaryl (optionally substituted with R³), aryl-C₂₋₆ arkenyl-R⁵, heteroaryl (optionally substituted with R³), heteroaryl-C₁₋₆ alkyl-R³, cycloalkyl (optionally substituted with R³), and heterocycloalkyl (optionally substituted with R³);
R³ is C₁₋₆ alkyl, C₂₋₆ alkenyl-R⁵, halogen, CN, NO₂, N(R⁴)₂, OR⁴, C(=NOR⁶)R⁴, CON(R⁴)₂, COR⁴, CO₂R⁸, NR⁴R⁵, S(O)₀₋₂R⁶ or SO₂N(R⁴)₂;
R⁴ is H or a group selected from C₁₋₆ alkyl, aryl, C₁₋₆ alkyl-aryl, heteroaryl, C₁₋₆ alkyl-heteroaryl, cycloalkyl, C₁₋₆ alkyl-cycloalkyl, heterocycloalkyl and C₁₋₆ alkyl-heterocycloalkyl, wherein said group is optionally substituted with R⁶, COR⁶, SO₀₋₂R⁶, CO₂R⁶, OR⁶, CONR⁸R⁶, NR⁸R⁶, halogen, CN, SO₂NR⁸R⁶ or NO₂, and for each case of N(R⁴)₂ the R⁴ groups are the same or different or N(R⁴)₂ is heterocycloalkyl optionally substituted with R⁶, COR⁶, SO₀₋₂R⁶, CO₂R⁶, OR⁶, CONR⁸R⁶, NR⁸R⁶, halogen, CN, SO₂NR⁸R⁶ or NO₂;
R⁵ is COR⁴, CON(R⁴)₂, CO₂R⁶ or SO₂R⁶;
R⁶ is C₁₋₆ alkyl, aryl, C₁₋₆ alkyl-aryl, heteroaryl or C₁₋₆ alkyl-heteroaryl; and
R⁷ is OR⁴, COR⁴, CO₂R⁸, CON(R⁴)₂, NR⁴R⁵, S(O)₀₋₂R⁶, SO₂N(R⁴)₂, halogen, CN or cycloimidyl (optionally substituted with R⁸); and
R⁸ is H or C₁₋₆ alkyl;
and the salts, solvates, hydrates, N-oxides, protected amino, protected carboxy, or protected hydroxamic acid derivatives thereof.

Compounds of formula (I) are disclosed for the first time as having therapeutic utility. Compounds of formula (I) are new, except those wherein Y is OH and NB₂ is optionally substituted heterocycloalkyl and/or CR¹R² is optionally substituted cycloalkyl or heterocycloalkyl.

Combinations of su stituents and/or variables are only permissible if such combinations result in stable compounds.

### Description of the Invention

Preferred compounds of the invention are those wherein any one or more of the following apply:
X is SO₂;
B is not H;
B is optionally substituted C₁₋₆ alkyl, cycloalkyl, heterocycloalkyl, C₁₋₆ alkyl-aryl or C₁₋₆ alkyl-heteroaryl;
B-N-B is an optionally substituted heterocycloalkyl ring;
B-N-B is a heterocycloalkyl ring substituted with =NOR⁴;
R¹ is optionally substituted C₁₋₆ alkyl, C₁₋₆ alkyl-heteroaryl, C₁₋₆ alkyl-aryl or C₁₋₆ alkyl-heterocycloalkyl;
CR¹R² is the said optionally substituted cycloalkyl or heterocycloalkyl ring;
R³ is C₁₋₆ alkyl, C₂₋₆ alkenyl-R⁵, halogen, CN, NO₂, N(R⁴)₂, OR⁴, COR⁴, NR⁴R⁵, S(O)₀₋₂R⁶ or SO₂N(R⁴)₂; and
R⁷ is CON(R⁴)₂, NR⁴R⁵, SO₂N(R⁴)₂ or cycloimidyl.

It will be appreciated that the compounds according to the invention can contain one or more asymmetrically substituted carbon and sulfur atoms. The presence of one or more of these asymmetric centres in a compound of formula (I) can give rise to stereoisomers, and in each case the invention is to be understood to extend to all such stereoisomers, including enantiomers and diastereomers, and mixtures including racemic mixtures thereof.

It will further be appreciated that the compounds according to the invention may contain an oxime. This oxime can give rise to geometrical isomers, and in each case the invention is to be understood to extend to all such isomers and mixtures thereof.

As used in this specification, alone or in combination, the term "C₁₋₆ alkyl" refers to straight or branched chain alkyl moiety having from one to six carbon atoms, including for example, methyl, ethyl, propyl, isopropyl, butyl, *tert*-butyl, pentyl, hexyl and the like.

The term "C₂₋₆ alkenyl" refers to a straight or branched chain alkyl moiety having two to six carbon atoms and having in addition one double bond, of either E or Z stereochemistry where applicable. This term would include for example, vinyl, 1-propenyl, 1- and 2- butenyl, 2- methyl-2-propenyl etc.

The term "cycloalkyl" refers to a saturated alicyclic moiety having from three to six carbon atoms and which is optionally benzofused at any available position. This term includes for example cyclopropyl, cyclobutyl, cyclopentyl, cyclohexyl, indanyl and tetrahydronaphthyl.

The term "heterocycloalkyl" refers to a saturated heterocyclic moiety having from three to six carbon atoms and one or more heteroatoms selected from N, O, S and oxidised versions thereof, and which is optionally benzofused at any available position. This term includes, for example, azetidinyl, pyrrolidinyl, tetrahydrofuranyl, piperidinyl, indolinyl and tetrahydroquinolinyl.

The term "cycloalkenyl" refers to an alicyclic moiety having from three to six carbon atoms and having in addition one double bond. This term includes, for example, cyclopentenyl and cyclohexenyl.

The term "heterocycloalkenyl" refers to an alicyclic moiety having from three to six carbon atoms and one or more heteroatoms selected from N, O, S and oxidised versions thereof, and having in addition one double bond. This term includes, for example, dihydropyranyl.

The term "aryl" refers to an aromatic carbocyclic radical having a single ring or two condensed rings. This term includes, for example phenyl or naphthyl.

The term "heteroaryl" refers to aromatic ring systems of five to ten atoms of which at least one atom is selected from O, N and S, and includes, for example, furanyl, thiophenyl, pyridyl, indolyl, quinolyl and the like.

The term "cycloimidyl" refers to a saturated ring of five to ten atoms containing the atom sequence -C(=O)NC(=O)-. The ring may be optionally benzofused at any available position. Examples include succinimidoyl, phthalimidoyl and hydantoinyl.

The term "benzofused" refers to the addition of a benzene ring sharing a common bond with the defined ring system.

The term "optionally substituted" means optionally substituted with one or more of the groups specified, at any available position or positions.

The term "halogen" means fluorine, chlorine, bromine or iodine.

The terms "protected amino", "protected carboxy" and "protected hydroxamic acid" mean amino, carboxy and hydroxamic acid groups which can be protected in a manner familiar to those skilled in the art. For example, an amino group can be protected by a benzyloxycarbonyl, *tert*-butoxycarbonyl, acetyl or like group, or may be in the form of a phthalimido or like group. A carboxyl group can be protected in the form of an ester such as the methyl, ethyl, benzyl or *tert*-butyl ester. A hydroxamic acid may be protected as either N or O-substituted derivatives, such as O-benzyl or O-*tert*-butyldimethylsilyl.

Salts of compounds of formula (I) include pharmaceutically-acceptable salts, for example acid addition salts derived from inorganic or organic acids, such as hydrochlorides, hydrobromides, p-toluenesulphonates, phosphates, sulphates, perchlorates, acetates, trifluoroacetates, propionates, citrates, malonates, succinates, lactates, oxalates, tartrates and benzoates.

Salts may also be formed with bases. Such salts include salts derived from inorganic or organic bases, for example alkali metal salts such as magnesium or calcium salts, and organic amine salts such as morpholine, piperidine, dimethylamine or diethylamine salts.

When the "protected carboxy" group in compounds ofthe invention is an esterified carboxyl group, it may be a metabolically-labile ester of formula CO₂R⁹ where R⁹ may be an ethyl, benzyl, phenethyl, phenylpropyl, α- or β-naphthyl, 2,4-dimethylphenyl, 4-*tert* butylphenyl, 2,2,2-trifluoroethyl, 1-(benzyloxy)benzyl, 1-(benzyloxy)ethyl, 2-methyl-1-propionyloxypropyl, 2,4,6-trimethylbenzyloxymethyl or pivaloylmethyl group.

Compounds of the general formula (I) may be prepared by any suitable method known in the art and/or by the following processes.

It will be appreciated that, where a particular stereoisomer of formula (I) is required, the synthetic processes described herein may be used with the appropriate homochiral starting material and/or isomers maybe resolved from mixtures using conventional separation techniques (e.g. HPLC).

The compounds according to the invention may be prepared by the following process. In the description and formulae below the groups R¹, R², R³, R⁴, R⁵, R⁶, R⁷, R⁸, R⁹, B, X and Y are as defined above, except where otherwise indicated. It will be appreciated that functional groups, such as amino, hydroxyl or carboxyl groups, present in the various compounds described below, and which it is desired to retain, may need to be in protected form before any reaction is initiated. In such instances, removal of the protecting group may be the final step in a particular reaction. Suitable protecting groups for such functionality will be apparent to those skilled in the art. For specific details see Greene *et al*, "Protective Groups in Organic Synthesis", Wiley Interscience.

A process for preparing compounds of general formula (I) comprises acylating an amine of formula B₂NH (II) with an acylating agent of formula Z-X-(CH₂)ₘ-CR¹R²-COY (III) wherein Z represents a suitable leaving group (e.g. a halogen such as bromine), and Y is OH or NHOH or a protected form thereof. This reaction may be performed in an inert solvent such as tetrahydrofuran, in the presence of an organic or inorganic base.

Acylating agents of formula (III) where X = SO₂ may be prepared from R¹⁰S-(CH₂)ₘ-CR¹R²-COY (IV), where R¹⁰ is H or a suitable labile group such as acetyl, by treatment with chlorine in an appropriate solvent such as water at an appropriate temperature such as 0°C. Acylating agents of formula (III) where X = SO may also be prepared from compound (IV) by treatment with SO₂Cl₂ and acetic anhydride in an appropriate solvent such as dichloromethane at an appropriate temperature such as 0°C.

Sulfanyl compounds of formula (IV) are readily prepared by alkylation of a compound R¹⁰SH with an alkylating agent of the form Z^{A}-(CH₂)ₘ-CR¹R²-COY (V), where Z^{A} is a leaving group (e.g. a halogen such as bromine, or an alkylsulfonate ester such as methanesulfonate). Many compounds of formula (V) are available commercially, or may be prepared by standard chemistry known to those skilled in the art from materials available commercially.

Compounds of formula (V) where m=1 may be prepared from compounds of formula HOCH₂CR¹R²COY (VI). Thus, for example, a compound of formula (V) where Z^{A} is methanesulfonate may be prepared by treatment of a compound of formula (VI) with methanesulfonyl chloride in the presence of an organic base such as triethylamine in an inert solvent such as dichloromethane.

Compounds of formula (VI) may be prepared by the reduction of compounds of formula R¹¹O₂CCR¹R²COY (VII), where COOR¹¹ represents a suitable ester, e.g an ethyl ester. Suitable conditions for the reduction comprise the use of diisobutylaluminium hydride in toluene at -40°C.

Compounds of formula (VII) may be prepared by the sequential alkylation of, for example, diethyl malonate, with alkylating agents of formula R¹-Z^{A}(VIII) and R²-Z^{A} (IX), wherein Z^{A} is as defined above, followed by hydrolysis under basic conditions. Many alkylating agents of formula (VIII) or (IX) are available commercially or may be prepared from materials available commercially by methods known to those skilled in the art.

Compounds of formula (IV) where m = 1 and R² = H may also be prepared by the reaction of a compound R¹⁰SH with an acrylate of the form H₂C=CR¹CO₂H (X). Compounds of formula (X) may be prepared by the Mannich reaction (i.e. with paraformaldehyde and piperidine in a suitable organic solvent, such as 1,4-dioxane) on a dicarboxylic acid of general formula HO₂C-CHR¹-CO₂H (XI). This reaction involves an eliminative decarboxylation step, resulting in the formation of an α,β-unsaturated carboxylic acid (i.e. where Y=OH) directly.

Dicarboxylic acids of formula (XI) may be prepared by the alkylation of, for instance, diethyl malonate with an alkylating agent of formula R¹-Z^{A} (VIII), wherein Z^{A} is as defined above, followed by hydrolysis under basic conditions.

Amines of the structure depicted in formula (II) are commercially available or may be prepared by standard aromatic, heteroaromatic or other chemistry known to those skilled in the art, from materials available commercially.

Compounds of formula (I) may also be prepared by interconversion of other compounds of formula (I). Thus, for example, a compound of formula (I) wherein R¹ is a C₁₋₆ alkyl group may be prepared by hydrogenation (using palladium on carbon in suitable solvent, such as an alcohol, e.g. ethanol) of a compound of formula (I) wherein R¹ is a C₂₋₆ alkenyl group. Similarly, a compound of formula (I) where X=SO₂ may be prepared from a compound of formula (I) where X=SO by oxidation with, for example sodium periodate and ruthenium chloride trihydrate in an appropriate solvent, for example acetonitile-tetrachloromethane-water. Acids of general formula (I) (Y=OH) may be converted to hydroxamic acids (Y=NHOH) using methods known to those skilled in the art.

Any mixtures of final products or intermediates obtained can be separated on the basis of the physico-chemical differences of the constituents, in known manner, into the pure final products or intermediates, for example by chromatography, distillation, fractional crystallization, or by formation of a salt if appropriate or possible under the circumstances.

The compounds according to the invention exhibit *in vitro* inhibiting activities with respect to the stromelysins, collagenases and gelatinases. Compounds according to the invention may also exhibit *in vitro* inhibition of membrane shedding events known to be mediated by metalloproteinases, for example, TNF release, TNF receptor shedding, IL-6 receptor shedding, IL- 1 receptor shedding, CD23 shedding and L-selectin shedding. The activity and selectivity of the compounds may be determined by use of the appropriate enzyme inhibition test, for example as described in Examples A-M of WO 98/05635, or by the following assay for the inhibition of CD23 shedding.

The potency of compounds of general formula (I) to act as inhibitors of the shedding of CD23 is determined using the following procedure: a 100 µM solution of the compound being tested, or dilutions thereof, is incubated at 37°C in an atmosphere of 5% CO₂ with RPMI 8866 cells, which shed CD23 spontaneously without stimulation. After 1 h, the cells are removed by centrifugation and the supernatant assayed for levels of sCD23 using an ELISA kit available commercially. The activity in the presence of 0.1 mM inhibitor, or dilutions thereof, is compared to activity in a control devoid of inhibitor and the results reported as that inhibitor concentration effecting 50% inhibition of the shedding of CD23.

This invention also relates to a method of treatment for patients (including man and/or mammalian animals raised in the dairy, meat or fur industries or as pets) suffering from disorders or diseases which can be attributed to MMPs as previously described, and more specifically, a method of treatment involving the administration of the matrix metalloproteinase inhibitors of formula (I) as the active constituents.

Accordingly, the compounds of formula (I) can be used among other things in the treatment of osteoarthritis and rheumatoid arthritis, and in diseases and indications resulting from the over-expression of these matrix metalloproteinases such as found in certain metastatic tumour cell lines.

As mentioned above, compounds of formula (I) are useful in human or veterinary medicine since they are active as inhibitors of TNF and MMPs. Accordingly in another aspect, this invention concerns:
a method of management (by which is meant treatment of prophylaxis) of disease or conditions mediated by TNF and/or MMPs in mammals, in particular in humans, which method comprises administering to the mammal an effective, amount of a compound of formula (I) above, or a pharmaceutically acceptable salt thereof; and
a compound of formula (I) for use in human or veterinary medicine, particularly in the management (by which is meant treatment or prophylaxis) of diseases or conditions mediated by TNF and/or MMPs; and
the use of a compound of formula (I) in the preparation of an agent for the management (by which is meant treatment or prophylaxis) of diseases or conditions mediated by TNF and/or MMPs.

The disease or conditions referred to above include inflammatory diseases, autoimmune diseases cancer, cardiovascular diseases, diseases involving tissue breakdown such as rheumatoid arthritis, osteoarthritis, osteoporosis, neurodegeneration, Alzheimer's disease, stroke, vasculitis, Crohn's disease, ulcerative colitis, multiple sclerosis, periodontitis, gingivitis and those involving tissue breakdown such as bone resorption, haemorrhage, coagulation, acute phase response, cachexia and anorexia, acute infections, HIV infections, fever, shock states, graft versus host reactions, dermatological conditions, surgical wound healing, psoriasis, atopic dermatitis, epidermolysis bullosa, tumour growth, angiogenesis and invasion by secondary metastases, ophthalmological disease, retinopathy, corneal ulceration, reperfusion injury, migraine, meningitis, asthma, rhinitis, allergic conjunctivitis, eczema, anaphylaxis, restenosis, congestive heart failure, endometriosis, atherosclerosis, endosclerosis and aspirin-independent anti-thrombosis.

Compounds of formula (I) may also be useful in the treatment of pelvic inflammatory disease (PID), age-related macular degeneration and cancer-induced bone resorption. Further, they can be used in the treatment of lung diseases, e.g. selected from cystic fibrosis, adult respiratory distress syndrome (ARDS), emphysema, bronchitis obliterans-organising pneumonia (BOOP), idiopathic pulmonary fibrosis (PIF), diffuse alveolar damage, pulmonary Langerhan's cell granulamatosis, pulmonary lymphangioleiomyomatosis (LAM) and chronic obstructive pulmonary disease (COPD).

For the treatment of rheumatoid arthritis, osteoarthritis, and in diseases and indications resulting from the over-expression of matrix metalloendoproteinases such as found in certain metastatic tumour cell lines or other diseases mediated by the matrix metalloendoproteinases or increased TNF production, the compounds of formula (I) may be administered orally, topically, parenterally, by inhalation spray or rectally in dosage unit formulations containing non-toxic pharmaceutically acceptable carriers, adjuvants and vehicles. The term parenteral as used herein includes subcutaneous injections, intravenous, intramuscular, intrasternal injection or infusion techniques. In addition to the treatment of warm-blooded animals such as mice, rats, horses, cattle, sheep, dogs, cats etc, the compounds of the invention are effective in the treatment of humans.

The pharmaceutical composition containing the active ingredient may be in a form suitable for oral use, for example, as tablets, troches, lozenges, aqueous or oily suspensions, dispersible powders or granules, emulsions, hard or soft capsules, or syrups or elixirs. Compositions intended for oral use may be prepared according to any method known to the art for the manufacture of pharmaceutical compositions and such compositions may contain one or more agents selected from the group consisting of sweetening agents, flavouring agents, colouring agents and preserving agents in order to provide pharmaceutically elegant and palatable preparations. Tablets contain the active ingredient in admixture with non-toxic pharmaceutically acceptable excipients which are suitable for the manufacture of tablets. These excipients may be for example, inert diluents, such as calcium carbonate, sodium carbonate, lactose, calcium phosphate or sodium phosphate; granulating and disintegrating agents, for example corn starch, or alginic acid; binding agents, for example starch, gelatin or acacia, and lubricating agents, for example magnesium stearate, stearic acid or talc. The tablets may be uncoated or they may be coated by known techniques to delay disintegration and absorption in the gastointestinal tract and thereby provide a sustained action over a longer period. For example, a time delay material such as glyceryl monostearate or glyceryl distearate may be employed. They may also be coated by the techniques described in the US Patents 4,256,108; 4,166,452; and 4,265,874, to form osmotic therapeutic tablets for control release.

Formulations for oral use may also be presented as hard gelatin capsules where in the active ingredient is mixed with an inert solid diluent, for example calcium carbonate, calcium phosphate or kaolin, or as soft gelatin capsules wherein the active ingredient is mixed with water or an oil medium, for example peanut oil, liquid paraffin or olive oil. Aqueous suspensions contain the active materials in admixture with excipients suitable for the manufacture of aqueous suspensions. Such excipients are suspending agents, for example sodium carboxymethylcellulose, methylcellulose, hydroxy- propylmethylcellulose, sodium alginate polyvinyl-pyrrolidone, gum tragacanth and gum acacia; dispersing or wetting agents may be a naturally occurring phosphatide, for example lecithin, or condensation products of an alkylene oxide with fatty acids, for example polyoxyethylene stearate, or condensation products of ethylene oxide with long chain aliphatic alcohols, for example heptadecaethyleneoxycetanol, or condensation products of ethylene oxide with partial esters derived from fatty acids and a hexitol such a polyoxyethylene with partial esters derived from fatty acids and hexitol anhydrides, for example polyoxyethylene sorbitan monooleate. The aqueous suspensions may also contain one or more preservatives, for example ethyl, or n-propyl, p-hydroxybenzoate, one or more colouring agents, one or more flavouring agents, and one or more sweetening agents, such as sucrose or saccharin.

Oily suspensions may be formulated by suspending the active ingredient in a vegetable oil, for example arachis oil, olive oil, sesame oil or coconut oil, or in a mineral oil such as liquid paraffin. The oily suspensions may contain a thickening agent, for example beeswax, hard paraffin or cetyl alcohol. Sweetening agents such as those set forth above, and flavouring agents may be added to provide a palatable oral preparation. These compositions may be preserved by the addition of an anti-oxidant such as ascorbic acid.

Dispersible powders and granules suitable for preparation of an aqueous suspension by the addition of water provide the active ingredient in admixture with a dispersing or wetting agent, suspending agent and one or more preservatives. Suitable dispersing or wetting agents and suspending agents are exemplified, for example sweetening, flavouring and colouring agents, may also be present.

The pharmaceutical compositions of the invention may also be in the form of oil-in-water emulsions. The oily phase may be a vegetable oil, for example olive oil or arachis oil, or a mineral oil, for example liquid paraffin or mixtures of these. Suitable emulsifying agents may be naturally- occurring gums, for example gum acacia or gum tragacanth, naturally-occurring phosphatides, for example soya bean, lecithin, and esters or partial esters derived from fatty acids and hexitol anhydrides, for example sorbitan monooleate and condensation products of the said partial esters with ethylene oxide, for example polyoxyethylene sorbitan monooleate. The emulsions may also contain sweetening and flavouring agents.

Syrups and elixirs may be formulated with sweetening agents, for example glycerol, propylene glycol, sorbitol or sucrose. Such formulations may also contain a demulcent, a preservative and flavouring and colouring agents. The pharmaceutical compositions may be in the form of a sterile injectable aqueous or oleagenous suspension. This suspension may be formulated according to the known art using those suitable dispersing or wetting agents and suspending agents which have been mentioned above. The sterile injectable preparation may also be in a sterile injectable solution or suspension in a non-toxic parenterally-acceptable diluent or solvent, for example as a solution in 1,3-butane diol. Among the acceptable vehicles and solvents that may be employed are water, Ringer's solution and isotonic sodium chloride solution. In addition, sterile, fixed oils are conventionally employed as a solvent or suspending medium. For this purpose any bland fixed oil may be employed including synthetic mono- or diglycerides. In addition, fatty acids such as oleic acid find use in the preparation of injectables.

The compounds of formula (I) may also be administered in the form of suppositories for rectal administration ofthe drug. These compositions can be prepared by mixing the drug with a suitable non-irritating excipient which is solid at ordinary temperatures but liquid at the rectal temperature and will therefore melt in the rectum to release the drug. Such materials are cocoa butter and polyethylene glycols.

For topical use, creams, ointments, jellies, solutions or suspensions, etc containing the compounds of Formula (I) are employed. For the purposes of this specification, topical application includes mouth washes and gargles.

Dosage levels of the order of from about 0.05 mg to about 140 mg per kilogram of body weight per day are useful in the treatment of the above- indicated conditions (about 2.5 mg to about 7 g per patient per day). For example, inflammation may be effectively treated by the administration of from about 0.01 to 50 mg of the compound per kilogram of body weight per day (about 0.5 mg to about 3.5 g per patient per day).

The amount of active ingredient that may be combined with the carrier materials to produce a single dosage form will vary depending upon the host treated and the particular mode of administration. For example, a formulation intended for the oral administration of humans may vary from about 5 to about 95% of the total composition. Dosage unit forms will generally contain between from about 1 mg to about 500 mg of active ingredient.

It will be understood, however, that the specific dose level for any particular patient will depend upon a variety of factors including the activity of the specific compound employed, the age, body weight, general health, sex, diet time of administration, route of administration, rate of excretion, drug combination and the severity of the particular disease undergoing therapy.

The following Examples illustrate the invention.

In the Examples, the following abbreviations are used:
- TNFα: Tumour Necrosis Factor α
- LPS: Lipopolysaccharide
- ELISA: Enzyme-linked immunosorbant assay
- EDC: 1-Ethyl-2-dimethylaminopropylcarbodiimide
- RT: Room Temperature

### Intermediate 1 3-Iodo-1-(3,4,4-trimethyl-2,5-dioxoimidazolidin-1-yl)-propane

Sodium hydride (2.2 g) was added to a solution of 3,4,4-trimethylhydantoin (7.1 g) in dimethylformamide (50 ml) at room temperature and the mixture was stirred for 1 h. 3-Chloro-1-bromopropane (4.9 ml) was then added and the solution was stirred overnight. The mixture was then poured into water (300 ml) and extracted with diethyl ether; the ether layer was then dried (MgSO₄) and evaporated and the residue was dissolved in acetone (100 ml) to which was added sodium iodide (10 g). The mixture was heated at reflux for 18 h, then evaporated and the residue was dissolved in diethyl ether and washed with water, then dried (MgSO₄) and evaporated to give the *title compound* (11 g, 70%) as a brown oil.
R_{f} 0.85 (diethyl ether)

### Intermediate 2 Dibenzyl (3-(3,4,4-Trimethyl-2,5-dioxoimidazolidin-1-yl)-propyl)malonate

A solution of dibenzyl malonate (5.7 g) in anhydrous tetrahydrofuran (200 ml) was treated at 0°C with sodium hydride (60% dispersion in mineral oil, 3.1 g). The mixture was allowed to warm to room temperature and stirred for 30 minutes under nitrogen. The mixture was then treated with a solution of intermediate 1 (6.2 g) in tetrahydrofuran (100 ml) and heated at reflux for 12 hours. The reaction was then cooled, filtered, and the filtrate evaporated *in vacuo*. The residue was partitioned between ethyl acetate (200 ml) and saturated aqueous ammonium chloride (150 ml). The organic layer was washed with water (100 ml), brine (100 ml), dried (MgSO₄), filtered and the filtrate evaporated *in vacuo*. The residue was purified by column chromatography on silica gel eluting with 20% ethyl acetate in hexane to yield the title compound (7.1 g, 80%) as a colourless oil.
R_{f} 0.53 (diethyl ether)

### Intermediate 3 2-Methylene-5-(3,4,4-trimethyl-2,5-dioxoimidazolidin-1-yl)-pentanoic Acid

A solution of intermediate 2 (10 g) in dioxane (200 ml) was treated with 10% palladium on charcoal (1.7 g) and hydrogenated at atmospheric pressure until the hydrogen uptake had ceased. The catalyst was removed by filtration through Celite® and the filtrate treated with piperidine (3.2 g) at room temperature. After 30 minutes the reaction was treated with formaldehyde (37% solution in water, 15 ml), stirred for two hours at room temperature and then heated at 80 °C for two hours. The mixture was cooled, the solvent removed *in vacuo* and the residue partitioned between ethyl acetate (200 ml) and 10% aqueous citric acid (100ml). The organic layer was washed with water (100 ml), brine (100 ml), dried over magnesium sulfate, and filtered, and the filtrate evaporated *in vacuo*. The residue was purified by column chromatography on silica gel eluting with 40% ethyl acetate in hexane to yield the title compound (5.40 g, 95%) as a colourless oil.
R_{f} 0.4 (ethyl acetate)

### Intermediate 4 2-Methylene-3-methylbutanoic acid

Morpholine (39.4 g) was added to a solution of isopropylmalonic acid (60.1 g) in water (300 ml) and acetic acid (47 ml). The mixture was stirred for 20 min, then a solution of aqueous formaldehyde (37% aq, 18.54 g) was added. The mixture was stirred at room temperature overnight, then heated at 80°C for 2 h and recooled to room temperature. The mixture was filtered, basified with sodium bicarbonate and washed with dichloromethane (100 ml). The pH was adjusted to 4 with dilute HCl, then the mixture was extracted with dichloromethane (3 x 200 ml). the organic extracts were combined and washed with water and brine, dried (Mg SO₄) and then evaporated to give the title compound as colourless oil (32 g).
R_{f} 0.45 (EtOAc).

### Intermediate 5 2-Bromomethyl-5-(3,4,4-trimethyl-2,5-dioxoimidazolidin-1-yl)-pentanoic Acid

Intermediate 3 (5.4 g) was treated with 45% hydrogen bromide in acetic acid (50 ml) at room temperature. After three hours the solution was poured into water (300 ml) and the product extracted with ethyl acetate (3 x 100ml). The organic extracts were combined, washed with water (100 ml), brine (100 ml), dried over magnesium sulfate, and filtered, and the filtrate evaporated *in vacuo*. The residue was dried by azeotrope with toluene (2 x 10ml) to yield the title compound (4.0 g, 56 %) as a viscous oil.
R_{f} 0.35 (ethyl acetate)

Similarly prepared was

### Intermediate 6 2-Bromomethyl-3-methylbutanoic Acid

From intermediate 4 (32 g), as colourless liquid (52.3 g).
R_{f} 0.43 (EtOAc).

### Intermediate 7 Methyl 2-Bromomethyl-5-(3,4,4-trimethyl-2,5-dioxoimidazolidin-1-yl)pentanoate

Acetyl chloride (15 ml) was added to ice cold methanol (200 ml) dropwise over 20 min, then a solution of intermediate 5 (3.5 g) in methanol (20 ml) was added and the solution was stirred at room temperature for 3 h. The mixture was then evaporated *in vacuo* and the residue dissolved in dichloromethane (100 ml) and washed with saturated sodium bicarbonate solution and brine. The solvent was dried and evaporated to give the title compound (2.05 g) as pale yellow oil.
R_{f} 0.65 (ether).

### Intermediate 8 tert-Butyl 2-Bromomethyl-5-(3,4,4-trimethyl-2,5-dioxoimidazolidin-1-yl)pentanoate

Isobutylene (100 ml) was added to a solution of the intermediate 5 (36.1 g) and sulfuric acid (1.2 ml) in dichloromethane (110 ml) at -78°C in a Parr pressure reactor. The vessel was sealed and the mixture stirred at room temperature for 24 h, then cooled to -5°C and the solution was removed and washed with saturated sodium carbonate and water, then dried (MgSO₄) and evaporated to give the *title compound* as a colourless oil (35.9 g).
R_{f} 0.73 (ether).

Similarly prepared was

### Intermediate 9 tert-Butyl 2-Bromomethyl-3-methylbutanoate

From intermediate 6 (52.3 g), as colourless liquid (64.0 g).
R_{f} 0.72 (EtOAc).

### Intermediate 10 Methyl 2-Acetylsulfanylmethyl-5-(3,4,4-trimethyl-2,5-dioxoimidazolidin-1-yl)pentanoate

Potassium thiolacetate (1.2 g) was added to a solution of intermediate 7 (1.5 g) in dimethylformamide (20 ml) at room temperature and the mixture was then heated at 100°C for 3 h. The mixture was added to water and extracted with ether (3 x 50 ml). The solvent was washed with sodium bicarbonate solution, water and brine, the dried (MgSO₄) and evaporated to give a brown oil. This was purified by silica gel column chromatography, eluting with ether, to give the title compound (1.2 g) as an amber oil.
R_{f} 0.55 (ether)

Similarly prepared were:

### Intermediate 11 tert-Butyl 2-Acetylsulfanylmethyl-5-(3,4,4-trimethyl-2,5-dioxoimidazolidin-1-yl)pentanoate

From intermediate 8 (35.9 g), as a yellow oil (34.5 g).
R_{f} 0.66 (ether).

### Intermediate 12 tert-Butyl 2-Acetylsulfanylmethyl-3-methylbutanoate

From intermediate 9 (64 g) as colourless oil (55.7 g).
R_{f} 0.68 (EtOAc).

### Intermediate 13 Methyl 2-(Chlorosulfonylmethyl)-5-(3,4,4-trimethyl-2,5-dioxoimidazolidin-1-yl)pentanoate

Chlorine gas was bubbled through a suspension of intermediate 10 (1.2 g) in 5% aqueous acetic acid (50 ml) at 0°C for 20 min. The suspension was stirred for 20 min, then placed under vacuum to remove excess chlorine for 5 min. The suspension was then extracted with dichloromethane (2 x 50 ml) and the solvent washed with water and brine. The organic layer was dried over magnesium sulfate and evaporated to give the title compound (1.10 g) as pale yellow oil, which slowly crystallised on standing.
R_{f} 0.45 (ether).

Similarly prepared were:

### Intermediate 14 tert-Butyl 2-Chlorosulfonylmethyl-5-(3,4,4-trimethyl-2,5-dioxo-imidazolidin-1-yl)pentanoate

From intermediate 11 (1.04 g) as a viscous pale yellow oil (0.86 g).
R_{f} 0.48 (ether).

### Intermediate 15 tert-Butyl 2-Chlorosulfonylmethyl-3-methylbutanoate

From intermediate 12 (50 g) as pale yellow oil (49.9 g).
R_{f} 0.54 (EtOAc).

### Intermediate 16 N-Methyl-2-phenoxyethylamine

This compound was prepared according to the method described by Grieco and Bahsas (*J. Org. Chem.*, 1987, **52**, 5747-5749) from 2-phenoxyethylamine (1.00 g), as a near colourless oil (0.88 g, 81%).
R_{f} 0.32 (94:5:1 dichloromethane/methanol/ammonium hydroxide).

### Intermediate 17 2-Hydroxymethylbenzofuran

To a stirred solution of benzofuran 2-carboxylic acid (2.50 g) in tetrahydrofuran (50 ml) at 0°C under nitrogen was added a solution of lithium aluminium hydride (1.0M; 7.7 ml) in tetrahydrofuran. Stirring was continued for 2 h, allowing the temperature to rise to RT. The mixture was diluted with diethyl ether (50 ml) and quenched with water (30 ml). The aqueous layer was separated and extracted with diethyl ether (2 x 25 ml). The combined extracts were washed with water (30 ml), sodium hydroxide solution (1M; 30 ml), water (30 ml), brine (30 ml) and dried (MgSO₄). Filtration and evaporation of solvents under reduced pressure yielded the title compound (1.11g, 49%) as a colourless oil.
R_{f} 0.29 (2:1 hexane/ethyl acetate).

### Intermediate 18 2-Chloromethylbenzofuran

To a stirred solution of intermediate 17 (1.10 g) in dichloromethane (50 ml) at RT was added pyridine (646 mg) and 4-toluenesulfonyl chloride (1.56 g). Stirring was continued for 72 h at RT and a further 24 h at reflux. The cooled solution was diluted with dichloromethane (50 ml) and washed with dilute hydrochloric acid (1M; 25 ml), water (2 x 25 ml), brine (25 ml) and dried (MgSO₄). Filtration and evaporation of solvents under reduced pressure and purification of the residue by silica gel column chromatography, eluting with 5:1 hexane/ethyl acetate, yielded the title compound (337 mg, 27%) as a colourless oil.
R_{f} 0.49 (2:1 hexane/ethyl acetate).

### Intermediate 19 N-Methyl Benzofuran-2-yl methylamine

This compound was prepared according to the method of Butera *et al* (*J. Med. Chem.,* 1991, **34**, 3212-3228) from intermediate 18 (330 mg), as a near colourless oil (883 mg, 8%).
R_{f} 0.30 (9:1 dichloromethane/methanol)

### Intermediate 20 Diethyl Tetrahydropyran-4,4-dicarboxylate

Diethyl malonate (32.0 g) was added to a solution of sodium ethoxide (1 equivalent) in ethanol and the solution was stirred for 30 min. 2-Bromoethylether (46.0 g) was then added and the mixture was stirred at reflux for 3 h. The mixture was then cooled, evaporated *in vacuo* and the residue partitioned between water and dichloromethane. The organic layer was separated and washed with water and brine, then dried (MgSO₄) and evaporated. The residue was then purified by flash column chromatography on silica gel, eluting with 4:1 hexanes/ether, to give the title compound (28.0 g) as colourless liquid.
R_{f} 0.33 (4:1 hexanes/ether).

### Intermediate 21 Ethyl 4-Hydroxymethyltetrahydropyran-4-carboxylate

A solution of di-isobutylaluminium hydride in toluene (82 mmol) was added to a solution of intermediate 20 (9.5 g) in toluene at -40°C over 30 min. The mixture was stirred for 1 h, then ethanol (100 ml) was added dropwise over 30 min. Sodium borohydride (2.0 g) was then added in small portions over 20 min, and the mixture stirred for 1 h. Saturated sodium sulfate (100 ml) was then added dropwise followed by ethyl acetate (200 ml). The mixture was vigorously stirred for 1 h, then filtered through Celite and the filtrate evaporated to give the title compound (5.6 g) as colourless liquid.
R_{f} 0.60 (EtOAc).

### Intermediate 22 Ethyl 4-(Methanesulfonyloxy)methyltetrahydropyran-4-carboxylate

Methanesulfonyl chloride (4.6 ml) was added to a solution of intermediate 21 (11.0 g) at 0°C in dichloromethane (30 ml), followed by triethylamine (8.0 ml). The mixture was stirred for 1 h, then washed with citric acid (5% aq, 30 ml), saturated sodium bicarbonate and brine. The organic layer was separated, and then dried (MgSO₄) and evaporated to give the title compound as colourless oil (15.2 g).
R_{f} 0.65 (ether).

### Intermediate 23 Ethyl 4-(Acetylsulfanylmethyl)tetrahydropyran-4-carboxylate

A solution of intermediate 22 (16.0 g), sodium iodide (0.2 g) and potassium thioacetate (12.0 g) in dimethylformamide (100 ml) was heated at 80°C for 6 h. The resulting black viscous mixture was then added to aqueous bicarbonate (300 ml) and extracted with ether. The ether layer was washed with water and brine, then dried (MgSO₄) and evaporated. The residue was purified by flash column chromatography on silica gel, eluting with 1:1 ether/hexanes, to give the title compound (6.5 g) as pale yellow oil.
R_{f} 0.45 (1:1 ether/hexanes)

### Intermediate 24 Ethyl 4-(Chlorosulfonyl)methyltetrahydropyran-4-carboxylate

Chlorine gas was bubbled through a suspension of intermediate 23 (3.2 g) in water (100 ml) and acetic acid (5 ml) at 0°C for 30 min. The yellow suspension was stirred at the same temperature for 30 min, then partially evaporated under vacuum and the aqueous residue extracted with dichloromethane (100 ml). The combined organic extracts were washed with iced-cold water and brine, then dried (MgSO₄) and evaporated to give the title compound (3.3 g) as colourless solid.
R_{f} 0.45 (ether).

### Intermediate 25 2-(4-Chlorophenoxy)-2,2-dimethylpropionyl Chloride

To a stirred solution of 2-(4-chlorophenoxy)-2,2-dimethylpropionic acid (5.00g) in dichloromethane (100 ml) and dimethylformamide (2 drops) at 0 °C under nitrogen was added oxalyl chloride (14.8 g). The mixture was stirred overnight with the temperature rising to RT. The mixture was evaporated under reduced pressure to dryness to yield the title compound (5.26g, 97%) as a brown liquid.
R_{f} 0.65 (5:1 hexane/ethyl acetate).

### Intermediate 26 2-(4-Chlorophenoxy)-2,2-dimethylpropionic Acid N-Methyl Amide

To a stirred 40 % aqueous solution of methylamine (20 ml) at 0 °C was added intermediate 25 (1.00g). The mixture was stirred for 30 mins and then the resulting precipitate was collected by filtration and dried *in vacuo* to yield the title compound (576mg, 59%)as a white solid.
R_{f} 0.47 (2:1 ethyl acetate/hexane).

### Intermediate 27 N-[2-(4-chlorophenoxy)-2,2-dimethylpropyl]-N-methylamine

To a stirred solution of intermediate 26 (300 mg) in tetrahydrofuran (10 ml) at 0°C under nitrogen was added a solution of borane-dimethyl sulphide in tetrahydrofuran (2M, 1 ml). The mixture was heated at reflux for 4 h then cooled and diluted with water (30 ml). The mixture was extracted with dichloromethane (3 x 20ml) and the combined extracts were washed with water (2 x 20 ml), brine (20 ml) and dried (MgSO₄). Filtration and evaporation of solvents *in vacuo* yielded a colourless oil. To a stirred solution of the oil in methanol (20 ml) at RT was added concentrated hydrochloric acid (3 ml). The mixture was stirred for 2.5 h then diluted with water (80 ml) and washed with hexane (2 x 20 ml). The aqueous mixture was basified (2M NaOH; pH 12) and extracted with dichloromethane (5 x 20 ml). The combined organic fractions were washed with water (2 x 20 ml), brine (20 ml) and dried (Mg SO₄). Filtration and evaporation of solvents *in vacuo* yielded the title compound (146mg, 58 %) as a colourless oil.
R_{f} 0.32(94:5:1 dichloromethane/methanol/ammonium hydroxide).

### Intermediate 28 N-(tert-Butyloxycarbonyl)-4-piperidinol

Was prepared according to the method of K.M. Wells *et al* (*Tetrahedron Lett*., 1996, *37(36)*, 6439-6442) as a pale yellow solid (46.5 g, 99%)
R_{f} 0.67 (EtOAc)

### Intermediate 29 4-(Piperidin-4-yloxy)pyridine, Bis(Trifluoroacetate)

Sodium hydride (1.8 g) was added to a solution of intermediate 28 (3.4 g) in dimethylformamide (15 ml), cooled in ice, and held under nitrogen. The suspension was then stirred at room temperature for 1 h, treated with 4-chloropyridine hydrochloride (2.7 g), and heated to 100°C for a further 2 h. After cooling, the mixture was partitioned between diethyl ether (100 ml) and water (100 ml). The organic phase was collected, washed with water (20 ml), saturated brine (20 ml), dried (Na₂SO₄) and evaporated to dryness *in vacuo*. The beige solid residue was recrystallised from diethyl ether to provide the intermediate ether as a crystalline pale brown solid (4.24 g). The recrystallised solid was then dissolved in dichloromethane (80 ml), treated with trifluoroacetic acid (20 ml), and stirred at room temperature for 16 h. The mixture was then concentrated under reduced pressure and co-evaporated with 50 % dichloromethane/hexane (20 ml) to provide the title compound as a beige solid (4.8 g, 70%).
R_{f} 0.11 (8% methanol/dichloromethane with trace triethylamine)

Similarly prepared were:

### Intermediate 30 4-(4-Cyanophenyloxy)piperidine, Trifluoroacetate

From intemediate 28 (3.0 g) and 4-fluorobenzonitrile (1.95 g), as a colourless solid (1.34 g, 28%)
Rf 0.1 (50% hexane and 0.1% AcOH in EtOAc)

### Intermediate 31 4-(4-Chlorophenyloxy)piperidine, Hydrochloride

Sodium hydride, as a 60% dispersion in mineral oil (2.11 g) was added to a solution of intermediate 28 (10.0 g) in anhydrous dimethylsulfoxide (200 ml). After stirring for 30 min at RT under nitrogen, potassium benzoate (8.46 g) was added, followed, after 20 min, by 1-chloro-4-fluorobenzene (6.90 g). The mixture was heated at 60°C for 2 h, then cooled to RT and diluted with water (600 ml) and the pH adjusted to 5. The solution was washed with hexane (100 ml), basified to pH 11, then extracted with ethyl acetate (3 x 100 ml). The combined ethyl acetate extracts were washed with saturated aqueous brine (50 ml), dried over sodium sulfate and evaporated, and then the residue was crystallised from hexane. The crystalline solid obtained was dissolved in a mixture of ethanol and dioxane (1:1, 150 ml) and saturated with hydrogen chloride gas. After 2 h at RT, the solution was purged with nitrogen gas and then concentrated under reduced pressure. The residue was dissolved in a minimum amount of methanol, and then diluted with ether. The precipitate which formed was collected by filtration and dried to give the title product (5.36 g, 41%) as a colourless solid.
R_{f} 0.35 (8% MeOH/dichloromethane)

### Intermediate 32 N-(tert-Butoxycarbonyl)-4-piperidone

This compounds was prepared according to the method of I. M. Labouta *et al*, *Eur. J. Med*. *Chem. Chim. Ther.,* 1982, **17**, 531-535, as a colourless solid (9.72 g, 98%).

### Intermediate 33 tert-Butyl 4-Hydroxyiminopiperidine-1-carboxylate

Hydroxylamine hydrochloride (2.95 g), sodium acetate (3.80 g) and intermediate 32 (7.70 g) were combined in ethanol (40 ml) and stirred at room temperature for 72 h. Water (100 ml) was added, and the mixture extracted with diethyl ether (2 x 50 ml). The combined organic extracts were washed with saturated sodium bicarbonate (2 x 30 ml), water (30 ml), saturated brine (30 ml), dried (MgSO₄) and concentrated *in vacuo* to provide the title compound (7.94 g, 96%) as a pale yellow solid.
R_{f} 0.30 (2% methanol/dichloromethane with trace triethylamine)

### Intermediate 34 tert-Butyl 4-(4-Cyanophenoxyimino)piperidine-1-carboxylate

Sodium hydride, as a 60% dispersion in mineral oil (0.103 g), was added to a solution of intermediate 33 (0.50g) in anhydrous tetrahydrofuran (30 ml), and the mixture stirred under nitrogen at room temperature. After 30 min, 4-fluorobenzonitrile was added as a solution in dry tetrahydrofuran (3 ml), and the mixture stirred for a further 24 h at room temperature. The solvents were then removed *in vacuo*, the residue azeotroped twice with toluene before purification by silica gel column chromatography, with 2% methanol/dichloromethane as eluent, to provide the title compound (0.57 g, 78%) as a pale yellow solid.
R_{f} 0.63 (2% methanol/dichloromethane with trace triethylamine)

Similarly prepared was:

### Intermediate 35 tert-Butyl 4-(4-Chlorobenzyloxyimino)piperidine-1-carboxylate

From intermediate 33 (0.80 g) and 4-chlorobenzyl bromide, as a colourless solid (0.54 g, 43%) after purification by silica gel column chromatography with 7% diethyl ether/dichloromethane as eluent.
R_{f} 0.50 (7% diethyl ether/dichloromethane)

### Intermediate 36 4-(4-Cyanophenoxyimino)piperidine, Trifluoroacetate Salt

Trifluoroacetic acid (1 ml) was added to a solution of intermediate 34 (0.56 g) in dichloromethane (9 ml). After stirring for 6 h at room temperature, the solvents were removed *in vacuo*. The residue was dissolved in dichloromethane (1 ml) and diluted with diethyl ether to induce crystallisation. The mixture was cooled, filtered and washed with diethyl ether to furnish the title compound (0.56 g, 96%) as a off-white solid.
R_{f} 0.28 (8% methanol/dichloromethane with trace triethylamine)

Similarly prepared was:

### Intermediate 37 4-(4-Chlorobenzyloxyimino)piperidine, Trifluoroacetate Salt

From intermediate 35 (0.54 g), as a white solid (0.49 g, 87%).
R_{f} 0.15 (3% methanol/dichloromethane with trace triethylamine)

### Example 1 Methyl 2-((2-Phenoxyethylsulfamoyl)methyl)-5-(3,4,4-trimethyl-2,5-dioxoimidazolidin-1-yl)pentanoate

To a stirred solution of intermediate 13 (500 mg) in dichloromethane (25 ml) at 0°C were added 2-phenoxyethylamine (186 mg) and triethylamine (137 mg). Stirring was continued for 18 h, with the temperature rising to RT. The reaction mixture was diluted with dichloromethane (50 ml) and washed with water (2 x 20 ml), hydrochloric acid (1M; 20 ml), water (20 ml), brine (20 ml) and then dried (MgSO₄). Filtration and evaporation of solvents *in vacuo* yielded the title compound (581 mg, 91%) as a colourless oil.
R_{f} 0.31 (3:1 ethyl acetate/hexane)
MS 467 (M⁺)

Similarly prepared were:

### Example 2 Methyl 2-((N-Methyl-(2-phenoxyethyl)sulfamoyl)methyl)-5-(3,4,4-trimethyl-2,5-dioxoimidazolidin-1-yl)pentanoate

From intermediate 16 (328 mg) and intermediate 13 (800 mg) as a colourless oil (874 mg, 83%).
R_{f} 0.27 (3:1 ethyl acetate/hexane)
MS 484 (M⁺)

### Example 3 Methyl 2-((N-Methyl-(2-(4-Chlorophenoxy)ethyl)-sulfamoyl)methyl)-5-(3,4,4-trimethyl-2,5-dioxoimidazolidin-1-yl)pentanoate

From 2-(4-chlorophenoxy)-*N*-methylethylamine (416 mg) and intermediate 13 (827 mg) as a colourless oil (955 mg, 82%).
R_{f} 0.35 (3:1 ethyl acetate/hexane).
MS 518 (M⁺)

### Example 4 tert-Butyl 2-((N-Methyl-(benzofuran-2-yl)methyl-sulfamoyl)-methyl)-5-(3,4,4-trimethyl-2,5-dioxoimidazolidin-1-yl)pentanoate

From intermediate 19 (180 mg) and intermediate 14 (459 mg) as a colourless oil (589 mg, 98%).
R_{f} 0.44 (2:1 ethyl acetate/hexane)
MS 536 (M⁺)

### Example 5 Ethyl 4-(4-(4-Chlorophenyl)piperazin-1-yl)sulfonylmethyl)-tetrahydropyran-4-carboxylate

4-Chlorophenylpiperazine dihydrochloride (7.3 g) and triethylamine (12 ml) were stirred in dichloromethane for 10 min, then the mixture was cooled in ice and a solution of intermediate 24 (6.9 g) in dichloromethane was added dropwise over 10 min. The mixture was stirred at 0°C for 3 h, washed with 2% aq. citric acid, saturated sodium bicarbonate and brine, dried (MgSO₄) and evaporated and the residue purified by chromatography (EtOAc) to give the title compound (8.60 g) as beige solid.
R_{f} 0.29 (ether)
MS 430 (M⁺).

Similarly prepared were:

### Example 6 Ethyl 4-(4-pyridyloxypiperidin-1-yl)sulfonylmethyltetrahydropyran-4-carboxylate

From intermediate 24 (1.0 g) and intermediate 29 (1.6 g) as colourless solid (1.45 g).
R_{f} 0.37 (6% MeOH/dichloromethane 1% NH₄OH)
MS 412 (M⁺)

### Example 7 Ethyl 4-(4-(4-Cyanophenoxy)piperidin-1-yl)sulfonylmethyltetrahydropyran-4-carboxylate

From intermediate 24 (1.0 g) and intermediate 30 (1.24 g) as beige solid (1.20 g). R_{f} 0.32 (ether)
MS 436 (M⁺)

### Example 8 Ethyl 4-(N-(2-(4-Chlorophenoxy)ethyl)-N-methylsulfamoylmethyl)tetrahydropyran-4-carboxylate

From intermediate 24 (685 mg) and *N*-(2-(4-chlorophenoxy)ethyl)-*N*-methylamine (500 mg) as a colourless oil (944 mg, 84%).
R_{f} 0.50 (2:1 ethyl acetate/hexane).
M⁺ 420.

### Example 9 Ethyl 4-(4-(4-Chlorophenoxy)piperidin-1-yl)sulfonylmethyltetrahydropyran-4-carboxylate

From intermediate 24 (3.28 g) and intermediate 31 (3.01 g) as a brown solid (4.47 g, 83%).
R_{f} 0.40 (ether)

### Example 10 tert-Butyl 2-(N-(2-(4-Chlorophenoxy)ethyl)-N-methylsulfamoylmethyl)-3-methylbutanoate

*N*-2-(4-Chlorophenoxy)ethyl*-N*-methylamine (300 mg) was added to a solution of intermediate 15 (438 mg) and triethylamine (1.2 ml) in dichloromethane (50 ml) at 0°C. The solution was stirred for 2 h, then washed with aqueous citric acid (5%, 50 ml), saturated bicarbonate solution (50 ml) and brine, dried (MgSO₄) and evaporated to give the title compound (469 mg, 69%) as a colourless oil.
R_{f} 0.43 (2:1 hexane/ethyl acetate).
MS 420 (M⁺)

Similarly prepared were:

### Example 11 tert-Butyl 2-[N-Methyl-N-(2-(4-Chlorophenoxy)-2,2-dimethylethyl)sulfamoyl]methyl-3-methylbutanoate

From intermediate 27 (366 mg) and intermediate 15 (464 mg) as a colourless oil (464 mg, 61%).
R_{f} 0.66(2:1 hexane/ethyl acetate).
MS 448 (M⁺)

### Example 12 tert-Butyl 2-(4-(4-Cyanophenoxyimino)piperidine-1-sulfonylmethyl)-5-(3,4,4-trimethyl-2,5-dioxoimidazolidin-1-yl)pentanoate

Triethylamine (0.30 ml) was added *via* syringe to an ice-cold solution of intermediate 14 (0.437 g) and intermediate 36 (0.350 g) in anhydrous dichloromethane (20 ml), and the mixture stirred under nitrogen for 3 days at RT. After evaporation of the mixture under reduced pressure, the residue was dissolved in diethyl ether (40 ml) and washed with 2.5% aqueous citric acid (2 x 10 ml), water (10 ml), saturated sodium bicarbonate (10 ml), water (10 ml), saturated brine (10 ml), dried (MgSO₄) and concentrated *in vacuo* to yield the title compound (0.434 g, 69%) as a white solid.
R_{f} 0.34 (diethyl ether)

Similarly prepared were:

### Example 13 tert-Butyl 2-(4-(4-Cyanophenoxyimino)piperidin-1-ylsulfonylmethyl)-3-methylbutanoic Acid

From intermediate 36(0.193 g) and intermediate 15 (0.181 g), as a colourless gum (0.087 g, 34%).
R_{f} 0.40 (2% diethyl ether/dichloromethane)

### Example 14 tert-Butyl 2-(4-(4-Chlorobenzyloxyimino)piperidin-1-ylsulfonylmethyl)-3-methylbutanoate

From intermediate 37 (0.48 g) and intermediate 15 (0.39 g), as a straw colourled gum (0.60 g, 92%).
R_{f} 0.40 (2% diethyl ether/dichloromethane)
MS 473 (MH⁺)

### Example 15 2-((2-Phenoxyethylsulfamoyl)methyl)-5-(3,4,4-trimethyl-2,5-dioxoimidazolidin-1-yl)pentanoic Acid

To a stirred solution of example 1 (555 mg) in dioxane (25 ml) in an ice-salt bath was added a solution of lithium hydroxide (149 mg) in water (10 ml). Stirring was continued for 5 h then the mixture was diluted with water (50 ml) and extracted with diethyl ether (2 x 25 ml). The aqueous mixture was acidified with citric acid to pH 4, and then extracted with ethyl acetate (5 x 30 ml). The combined ethyl acetate extracts were washed with water (2 x 20 ml), brine (20 ml) and dried (MgSO₄). Filtration and evaporation of solvents *in vacuo* yielded the title compound (443 mg, 82 %) as a white foam.
R_{f} 0.39 (3:1 ethyl acetate/hexane)
MS 455 (M⁺)

Similarly prepared were:

### Example 16 2-((N-Methyl-N-(2-phenoxyethyl)sulfamoyl)methyl)-5-(3,4,4-trimethyl-2,5-dioxoimidazolidin-1-yl)pentanoic Acid

From example 2 (850 mg) as a colourless oil (642 mg, 78%).
R_{f} (2:1 ethyl acetate/hexane).
MS 470 (M⁺)

### Example 17 2-((N-Methyl-N-(2-(4-Chlorophenoxy)ethyl)sulfamoyl)-methyl)-5-(3,4,4-trimethyl-2,5-dioxoimidazolidin-1-yl)pentanoic Acid

From example 3 (947 mg) as a colourless oil (772 mg, 84%).
R_{f} 0.26 (3:1 ethyl acetate/hexane)
MS 504 (M⁺)

### Example 18 2-((N-Methyl-(benzofuran-2-yl)methylsulfamoyl)methyl)-5-(3,4,4-trimethyl-2,5-dioxoimidazolidin-1-yl)pentanoic Acid

To a stirred solution of example 4 (575 mg) in dichloromethane (18 ml) at 0 °C was added trifluoroacetic acid (2 ml). The mixture was stirred for 16 h, with the temperature rising to RT. The solvent was removed *in vacuo* and the residue azeotroped with toluene (2 x 50 ml) to yield the title compound (515 mg, 100%) as a colourless oil.
R_{f} 0.17 (2:1 ethyl acetate/hexane).
MS 480 (M⁺)

### Example 19 4-(4-(4-Chlorophenyl)piperazin-1-yl)sulfonylmethyl)-tetrahydropyran-4-carboxylic Acid

Lithium hydroxide (6 g) was added to a solution of example 5 (8.6 g) in methanol (150 ml) and water (100 ml) and the solution was heated under reflux for 4 h. The mixture was cooled to RT, evaporated to half volume under reduced pressure and then the solution was washed with ether. The aqueous phase was acidified with citric acid to pH 5 and extracted with dichloromethane (4 x 100 ml). The solvent was washed with brine, dried (MgSO₄) and evaporated to give the title compound (5.60 g, 70 %) as beige solid.
R_{f} 0.20 (EtOAc)
MS 402 (M⁺)

Similarly prepared were:

### Example 20 4-(4-Pyridyloxypiperidin-1-yl)sulfonylmethyl)-tetrahydropyran-4-carboxylic acid

From example 6 (1.45 g) as beige solid (0.20 g).
R_{f} 0.20 (10% MeOH/dichforomethane 1% AcOH)
MS 384 (M⁺)

### Example 21 4-(4-(4-Cyanophenoxy)piperidin-1-yl)sulfonylmethyltetrahydropyran-4-carboxylic acid

From example 7 (1.20 g) as colourless solid (0.25 g).
R_{f} 0.54 (EtOAc + 1% AcOH)
MS 408 (M⁺)

### Example 22 4-(N-(2-(4-Chlorophenoxy)ethyl)-N-methylsulfamoylmethyl)tetrahydropyran-4-carboxylic Acid

From example 8 (930mg) as a colourless solid (688mg, 79%).
R_{f} 0.30(2:1 ethyl acetate/hexane).
MS 392 (M⁺)

### Example 23 4-(4-(4-Chlorophenoxy)piperidin-1-yl)sulfonylmethyltetrahydropyran-4-carboxylic Acid

From example 9 (3.1 g) as colourless solid (2.6 g, 90%).
R_{f} 0.40 (ether)
MS 416 (M-1)

### Example 24 2-(N-(2-(4-Chlorophenoxy)ethyl)-N-methylsulfamoyl-methyl)-3-methylbutanoic Acid

A solution of example 10 (465mg) was dissolved in 30% trifluoruacetic acid in dichloromethane (50 ml) and the solution was stirred for 2 h. The solution was then evaporated under reduced pressure and the residue azeotroped to dryness with dichloromethane/hexanes (3 x 100 ml). The residue was dissolved in saturated sodium bicarbonate (50 ml) and washed with ether. The aqueous layer was acidified with citric acid and extracted with EtOAc (2 x 100 ml), the solvent dried (MgSO₄) and evaporated to give the title compound (403mg, 100%) as a colourless oil.
R_{f} 0.55 (2:1 ethyl acetate/hexane).
MS 363 (M⁺)

Similarly prepared were:

### Example 25 2-(N-Methyl-N-(2-(4-chlorophenoxy)-2,2-dimethylethyl)-sulfamoylmethyl)-3-methylbutanoic Acid

From example 11 (450mg) as a colourless oil (379mg, 97%).
R_{f} 0.68 (2:1 ethyl acetate/hexane).
MS 392 (M⁺)

### Example 26 2-(4-(4-Cyanophenoxyimino)piperidin-1-ylsulfonylmethyl)-5-(3,4,4-trimethyl-2,5-dioxo-imidazolidin-1-yl)pentanoic Acid

Trifluoroacetic acid (2 ml) was added to a solution of example 12 (0.434 g) in dichloromethane (18 ml), and the mixture stirred at room temperature. After 18 h., the reaction mixture was evaporated under reduced pressure, and the residue azeotroped twice with toluene. The resultant oil was purified by silica gel column chromatography, eluting with 4% methanol/dichloromethane, to provide the title compound (0.232 g, 59%) as a pale yellow gum.
R_{f} 0.22 (4% methanol/dichloromethane)
MS 534 (MH⁺)

Similarly prepared were:

### Example 27 2-(4-(4-Cyanophenoxyimino)piperidin-1-ylsulfonylmethyl)-3-methylbutanoic Acid

From example 13 (0.087 g), as a colourless solid (0.085 g, 100%).
R_{f} 0.49 (3% methanol/dichloromethane)
MS 394 (MH⁺)

### Example 28 2-(4-(4-Chlorobenzyloxyimino)piperidin-1-ylsulfonylmethyl)-3-methylbutanoic Acid

From example 14 (0.60 g), as a white solid (0.42 g, 80%).
R_{f} 0.50 (5% methanol/dichloromethane)
MS 417 (MH⁺)

### Example 29 2-((2-Phenoxyethylsulfamoyl)methyl)-5-(3,4,4-tri-methyl-2,5-dioxoimidazolidin-1-yl)pentanoic Acid N-Hydroxy Amide

To a stirred solution of example 15 (440 mg) in dichloromethane (20 ml) at 0 °C was added EDC (189 mg) and O*-tert*-butyldimethylsilylhydroxylamine (141 mg). Stirring was continued for 5 h, with the temperature rising to RT, and then the mixture was diluted with dichloromethane (50 ml). The mixture was washed with water (3 x 20 ml), brine (20 ml) and dried (MgSO₄). Filtration and evaporation of solvents under reduced pressure gave a colourless oil that was dissolved in dichloromethane (15 ml) and treated with a solution of hydrogen chloride in diethyl ether (1M; 3.0 ml). The mixture was stirred for 1 h before the solvents were removed *in vacuo* and the residue was purified by silica gel column chromatography eluting with 3:1 ethyl acetate/hexane to yield the title compound (264 mg, 57%) as a white solid.
R_{f} 0.32 (3:1 ethyl acetate/hexane).
MS 471 (M⁺)

Similarly prepared were:

### Example 30 2-((N-Methyl-(2-phenoxyethyl)sulfamoyl)methyl)-5-(3,4,4-trimethyl-2,5-dioxoimidazolidin-1-yl)pentanoic Acid N-Hydroxy Amide

From example 16 (500 mg) as a white solid (175 mg, 34%).
R_{f} 0.16 (ethyl acetate).
MS 485 (M⁺)

### Example 31 2-((N-Methyl-(2-(4-Chlorophenoxy)ethyl)sulfamoyl)-methyl)-5-(3,4,4-trimethyl-2,5-dioxoimidazolidin-1-yl)pentanoic Acid N-Hydroxy Amide

From example 17 (750 mg) as a white solid (332 mg, 43%).
R_{f} 0.18 (ethyl acetate).
MS 519 (M⁺)

### Example 32 2-((N-Methyl-(benzofuran-2-yl)methyl-sulfamoyl)methyl)-5-(3,4,4-trimethyl-2,5-dioxoimidazolidin-1-yl)pentanoic Acid N-Hydroxy Amide

From example 18 (250 mg) as a white solid (98 mg, 38%).
R_{f} 0.10 (9:1 ethyl acetate/methanol)
MS 495 (M⁺).

### Example 33 2-(N-Methyl-N-(2-(4-chlorophenoxy)ethyl)-sulfamoylmethyl)-3-methylbutanoic Acid N-Hydroxy Amide

From example 24 (400mg) as a white foam (386mg, 96%).
R_{f} 0.46 (3:1 ethyl acetate/hexane).
M⁺ 379.

### Example 34 2-(N-Methyl-N-(2-(4-chlorophenoxy)-2,2-dimethylethyl)sulfamoylmethyl)-3-methylbutanoic Acid N-Hydroxy Amide

From example 25 (370mg) as a white solid (141mg, 37%).
R_{f} 0.32 (3:1 ethyl acetate/hexane).
M⁺ 407

### Example 35 4-(4-(4-Chlorophenyl)piperazin-1-yl)sulfonylmethyl)-tetrahydropyran-4-carboxylic Acid N-Hydroxy Amide Hydrochloride

Oxalyl chloride (4 ml) was added to a suspension of example 19 (5.6 g) in dichloromethane (100 ml) at 0°C, followed by dimethylformamide (1 drop). The mixture was stirred for 1 h, then evaporated *in vacuo* and the residue azeotroped with dichloromethane/hexanes (3 x 100 ml). The crude product was dissolved in dichloromethane (50 ml) and triethylamine (5.80 ml) and *O*-TBDMS hyroxylamine (2.24 g) were added. The mixture was stirred for 3 h, then washed with water, aqueous sodium bicarbonate and brine, dried and evaporated. The crude product was dissolved in dry dichloromethane (100 ml) and HCl in ether (1M, 50 ml) was added dropwise. The mixture was vigorously stirred for 30 min, then the product collected by filtration and washed with ether (2 x 100 ml) to give the title compound (5.0 g) as colourless powder.
R_{f} 0.53 (10% MeOH/dichloromethane 1% NH₄OH)
MS 418 (M⁺)

Similarly prepared were:

### Example 36 4-(4-(4-Pyridyloxy)piperidin-1-yl)sulfonylmethyl)-tetrahydropyran-4-carboxylic Acid N-Hydroxy Amide Hydrochloride

From example 20(0.19 g) as white solid (0.12 g).
R_{f} 0.25 (7% MeOH/dichloromethane 1% NH₄OH)
MS 400 (M⁺)

### Example 37 4-(4-(4-Cyanophenoxy)piperidin-1-yl)sulfonylmethyltetrahydropyran-4-carboxylic Acid N-Hydroxy Amide

From example 21 (0.25 g) as white solid (5.5 mg).
R_{f} 0.32 (5% MeOH/dichloromethane)
MS 423 (M⁺).

### Example 38 4-(N-(2-(4-Chlorophenoxy)ethyl)-N-methylsulfamoylmethyl)tetrahydropyran-4-carboxylic Acid N-Hydroxy Amide

From example 22 (650 mg) as a colourless solid (412mg, 74%).
R_{f} 0.24 (ethyl acetate).
M⁺ 407.

### Example 39 4-(4-(4-Chlorophenoxy)piperidin-1-yl)sulfonylmethyltetrahydropyran-4-carboxylic Acid N-Hydroxy Amide

From example 23 (2.69 g) as colourless solid (2.40 g, 86 %).
R_{f} 0.22 (5% MeOH/dichloromethane)
MS 431 (M-1)

### Example 40 2-[4-(4-Cyanophenoxyimino)piperidin-1-ylsulfonylmethyl]-5-(3,4,4-trimethyl-2,5-dioxoimidazolidin-1-yl)pentanoic Acid N-Hydroxy Amide

EDC (0.102 g) was added to a suspension of O-*tert*-butyldimethylsilylhydroxylamine (0.066 g) and example 26 (0.218 g) in dichloromethane (20 ml), and the mixture stirred at room temperature for 18 h. The solvent was then evaporated under reduced pressure. The residue was dissolved in ethyl acetate (50 ml) and washed with water (2 x 10 ml), saturated sodium bicarbonate (20 ml), water (10 ml), saturated brine (10 ml) and dried (MgSO₄). The organic phase was then evaporated *in vacuo*, the residue dissolved in tetrahydrofuran (5 ml), cooled in an ice-bath, and treated with tetrabutylammonium fluoride (1.0 M solution in tetrahydrofuran; 0.45 ml). After 10 min, the reaction was quenched with acetic acid (0.5 ml) and partitioned between water (30 ml) and hexane (20 ml). The hexane was decanted and the aqueous phase reshaken with hexane (20 ml). The aqueous phase was then extracted with ethyl acetate (2 x 20 ml), and the combined organic extracts were washed with a 1:1:1 mixture of water, saturated sodium bicarbonate and saturated brine (15 ml). The organic layer was futher washed with saturated brine (15 ml), then dried (MgSO₄) and evaporated under reduced pressure to yield the title compound (0.185 g, 82%) as a white solid.
R_{f} 0.16 (5% methanol/dichloromethane)
MS 549 (MH⁺)

Similarly prepared was:

### Example 41 2-(4-(4-Cyanophenoxyimino)piperidin-1-ylsulfonylmethyl)-3-methylbutanoic Acid N-Hydroxy Amide

From example 27 (0.085 g), as a white solid (0.017 g, 22%).
R_{f} 0.13 (3% methanol/dichloromethane)
MS 409 (MH⁺)

### Example 42 2-(4-(4-Chlorobenzyloxyimino)piperidin1-ylsulfonylmethyl)-3-methylbutanoic Acid N-Hydroxy Amide

EDC (0.251 g) was added to a solution of O-*tert*-butyldimethylsilylhydroxylamine (0.148 g) and example 28 (0.42 g) in dichloromethane (40 ml), and the mixture stirred at room temperature for 4 h. The solvent was then evaporated under reduced pressure, and the residue was dissolved in ethyl acetate (60 ml) and washed with 1% citric acid (20 ml), water (20 ml), saturated sodium bicarbonate (20 ml), water (10 ml), saturated brine (15 ml) and dried (MgSO₄). The organic phase was then evaporated *in vacuo*, and the residue purified by silica gel column chromatography with 4% methanol/dichloromethane, pre-eluting the column with 4% methanol/5% triethylamine/dichloromethane, to give a white solid (0.41 g). The white solid was then dissolved in dichloromethane (20 ml) and treated with hydrogen chloride, as a 1.0 M solution in diethyl ether (3.0 ml). After 20 min. at room temperature, the solvents were removed and the residue triturated with a mixture of dichloromethane and diethyl ether to provide the title compound (0.294 g, 67%) as a colourless solid.
R_{f} 0.14(5% methanol/dichloromethane)
MS 432 (MH⁺)

### HPLC-MS Conditions

In the following experimentals descriptions, HPLC-MS was performed on a Hewlet Packard 1100 LC using a Phenomenex Luna C18, 50x2.1mm column at 35°C, running a solvent A: solvent B gradient of 95:5 to 35:65 in 4.70 min and to 0:100 in 1.50 min at a flow rate of 0.90 mL/min. Solvent A and solvent B are 95% water:5% acetonitrile 0.1% formic acid and 5% water: 95% acetonitrile 0.1% formic acid respectively. MS spectra were acquired at 1 cone voltage (30V), on a Micromass Quattro (triple quadrupole) instrument

### Example 43 2-((N-Ethyl-N-(2-methoxyethyl)-sulfamoylmethyl)-5-(3,4,4-trimethyl-2,5-dioxoimidazolidin-1-yl)-pentanoic Acid N-Hydroxy Amide

(2-Methoxyethyl)ethylamine (0.1 mmol) was added to a solution of intermediate 15 (0.1 mmol) and *N*-methylmorpholine (1.1 eq) in dichloromethane (1 ml) at 0°C in a scintillation vials and the solutions was stirred overnight at room temperature. The solutions was washed with 0.2 M HCl (1 ml) and saturated bicarbonate solution, then evaporated to dryness. The crude product was dissolved in 50% trifluoroacetic acid/dichloromethane (1 ml) and stirred overnight at room temperature, then evaporated to dryness and azeotroped with toluene. The crude acids were then taken up in dichloromethane, and then treated with one equivalent each of EDC, hydroxylamine hydrochloride and *N*-methylmorpholine for 18 h. The solution was then washed with water and aqueous sodium bicarbonate, and evaporated. The crude product was purified by preparative HPLC, to give the title compound as a colourless foam (10.8 mg)
HPLC Rₜ 5.32 min.
MS 436 (M⁺)

Similarly prepared were

### Example 44 2-(3,3-Diphenylpropylsulfamoylmethyl)-5-(3,4,4-trimethyl-2,5-dioxo-imidazolidin-1-yl)-pentanoic Acid N-Hydroxy Amide

From intermediate 15 and 3,3-diphenylpropylamine, as a white solid (14.3 mg).
HPLC Rₜ 6.70 min.
MS 544 (M⁺)

### Example 45 2-(Cyclohezylmethylsulfamoylmethyl)-5-(3,4,4-trimethyl-2,5-dioxoimidaozolidin-1-yl)-pentanoic Acid N-Hydroxy Amide

From intermediate 15 and cyclohexylmethylamine, as a beige solid (5.5 mg).
HPLC Rₜ 5.58 min.
MS 446 (M⁺)

### Example 46 2-(1-Methyl-3-phenylpropylsulfamoylmethyl)-5-(3,4,4-trimethyl-2,5-dioxoimidazolidin-1-yl)pentanoic Acid N-hydroxy Amide

From intermediate 15 and 1-methyl-3-phenylpropylamine, as a white solid (24.7 mg).
HPLC Rₜ 5.88 min.
MS 482 (M⁺)

### Example 47 2-((3-Phenylpropyl)sulfamoylmethyl)-5-(3,4,4-trimethyl-2,5-dioxo-imidazolidin-1-yl)-pentanoic Acid N-Hydroxy Amide

From intermediate 15 and 3-phenylpropylamine, as a beige solid (34.1 mg).
HPLC Rₜ 5.61 min.
MS 468 (M⁺)

### Example 48 2-((N-Methyl-N-octylsulfamoyl)methyl)-5-(3,4,4-trimethyl-2,5-dioxoimidazolidin-1-yl)-pentanoic Acid N-Hydroxy Amide

From intermediate 15 and octylmethylamine, as a colourless soild (19.1 mg)
HPLC Rₜ 7.42 min.
MS 476 (M⁺)

### Example 49 2-(N-(2-Cyanoethyl)-N-octylsulfamoylmethyl)-5-(3,4,4-trimethyl-2,5-dioxoimidazolidin-1-yl)-pentanoic Acid N-Hydroxy Amide

From intermediate 15 and (2-cyanoethyl)octylamine, as a beige solid (3.1 mg).
HPLC Rₜ 7.23 min.
MS 515 (M⁺)

### Example 50 2-(N-(2-(4-Chlorophenoxy)-1-methylethyl)-N-methylsulfamoylmethyl)-5-(3,4,4-trimethyl-2,5-dioxoimidazolidin-1-yl)-pentanoic Acid N-Hydroxy Amide

From intermediate 15 and N-(2-(4-Chlorophenoxy)-1-methylethyl))methylamine, as a beige solid (7.3 mg).
HPLC Rₜ 6.46 min.
MS 532 (M⁺)

### Example 51 2-(N-methyl-N-phenethylsulfamoylmethyl)-5-(3,4,4-trimethyl-2,5-dioxoimidazolidin-1-yl)-pentanoic Acid N-Hydroxy Amide

From intermediate 15 and N-(phenethyl)methyamine, as a white solid (13.2 mg).
HPLC Rₜ 5.22 min.
MS 468 (M⁺)

### Example 52 2-(N-(2-(2-Chlorophenoxy)ethyl)-N-methyl-sulfamoylmethyl)-5-(3,4,4-trimethyl-2,5-dioxoimidazolidin-1-yl)-pentanoic Acid N-Hydroxy Amide

From intermediate 15 and N-(2-(2-chlorophenoxy)ethyl)methylamine, as a brown solid (5.5 mg).
HPLC Rₜ 5.68 min.
MS 518 (M⁺)

### Example 53 2-(N-Ethyl-N-phenylsulfamoylmethyl)-5-(3,4,4-trimethyl-2,5-dioxo-imidazolidin-1-yl)-pentanoic Acid N-Hydroxy Amide

From intermediate 15 and N-ethylaniline, and a colourless solid (9.0 mg).
HPLC Rₜ 5.89 min.
MS 454 (M⁺)

### Example 54 2-(N-(4-Chlorophenyl)-N-methylsulfamoylmethyl)-5-(3,4,4-trimethyl-2,5-dioxoimidazolidin-1-yl)pentanoic Acid N-Hydroxy Amide

From intermediate 15 and N-(4-chlorophenyl)methylamine, as a white solid (3.2 mg).
HPLC Rₜ 5.69 min.
MS 474 (M⁺)

## Claims

1. A compound for therapeutic use, represented by formula (I): 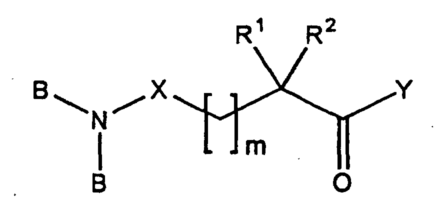 wherein
m is 0-2;
X is S(O)₁₋₂;
Y is OH or NHOH;
R¹ is H or a group (optionally substituted with R⁷) selected from C₁₋₆ alkyl, C₂₋₆ alkenyl, aryl, C₁₋₆ alkyl-aryl, heteroaryl, C₁₋₆ alkyl-heteroaryl, heterocycloalkyl, C₁₋₆ alkyl-heterocycloalkyl, cycloalkyl and C₁₋₆ alkyl-cycloalkyl, and R² is H or C₁₋₆ alkyl;
or CR¹R² is a cycloalkyl or heterocycloalkyl ring optionally substituted with R⁷ or a group (optionally substituted with R⁷) selected from C₁₋₆ alkyl, aryl, C₁₋₆ alkyl-aryl, heteroaryl and C₁₋₆ alkyl-heteroaryl;
each B is the same or different and is H or a group selected from C₁₋₆ alkyl-aryl, C₁₋₆ alkyl, cycloalkyl, C₁₋₆ alkyl-cycloalkyl, cycloalkenyl, heterocycloalkenyl, C₁₋₆ alkyl-heteroaryl, heterocycloalkyl, C₁₋₆ alkyl-heterocycloalkyl, aryl or heteroaryl, any of which groups is optionally substituted by a substituent selected from R³, C₁₋₆ alkyl-R³, C₂₋₆ alkenyl-R³, aryl (optionally substituted with R³), aryl-C₁₋₆ alkyl-R³, C₁₋₆ alkyl-aryl (optionally substituted with R³), C₁₋₆ alkyl-heteroaryl (optionally substituted with R³), aryl-C₂₋₆ alkenyl-R⁵, heteroaryl (optionally substituted with R³), heteroaryl-C₁₋₆ alkyl-R³, cycloalkyl (optionally substituted with R³) and heterocycloalkyl (optionally substituted with R³), provided that NB₂ is not NH₂,
or B-N-B is a heterocycloalkyl ring substituted with =O or =NOR⁴,
or, when neither R¹ nor R² is H, B-N-B is a heterocycloalkyl or heterocyclalkenyl ring optionally substituted by a substituent selected from R³, C₁₋₆ alkyl-R³, C₂₋₆ alkenyl-R³, aryl (optionally substituted with R³), aryl-C₁₋₆ alkyl-R³, C₁₋₆ alkyl-aryl (optionally substituted with R³), C₁₋₆ alkyl-heteroaryl (optionally substituted with R³), aryl-C₂₋₆ alkenyl-R⁵, heteroaryl (optionally substituted with R³), heteroaryl-C₁₋₆ alkyl-R³, cycloalkyl (optionally substituted with R³), and heterocycloalkyl (optionally substituted with R³);
R³ is C₁₋₆ alkyl, C₂₋₆ alkenyl-R⁵, halogen, CN, NO₂, N(R⁴)₂, OR⁴, C(=NOR⁶)R⁴, CON(R⁴)₂, COR⁴, CO₂R⁸, NR⁴R⁵, S(O)₀₋₂R⁶ or SO₂N(R⁴)₂;
R⁴ is H or a group selected from C₁₋₆ alkyl, aryl, C₁₋₆ alkyl-aryl, heteroaryl, C₁₋₆ alkyl-heteroaryl, cycloalkyl, C₁₋₆ alkyl-cycloalkyl, heterocycloalkyl and C₁₋₆ alkyl-heterocycloalkyl, wherein said group is optionally substituted with R⁶, COR⁶, SO₀₋₂R⁶, CO₂R⁶, OR⁶, CONR⁸R⁶, NR⁸R⁶, halogen, CN, SO₂NR⁸R⁶ or NO₂, and for each case of N(R⁴)₂ the R⁴ groups are the same or different or N(R⁴)₂ is heterocycloalkyl optionally substituted with R⁶ COR⁶, SO₀₋₂R⁶, CO₂R⁶, OR⁶, CONR⁸R⁶, NR⁸R⁶, halogen, CN, SO₂NR⁸R⁶ or NO₂;
R⁵ is COR⁴, CON(R⁴)₂, CO₂R⁶ or SO₂R⁶;
R⁶ is C₁₋₆ alkyl, aryl, C₁₋₆ alkyl-aryl, heteroaryl or C₁₋₆ alkyl-heteroaryl; and
R⁷ is OR⁴, COR⁴, CO₂R⁸, CON(R⁴)₂, NR⁴R⁵, S(O)₀₋₂R⁶, SO₂N(R⁴)₂, halogen, CN or cycloimidyl (optionally substituted with R⁸); and
R⁸ is H or C₁₋₆ alkyl;
and the salts, solvates, hydrates, N-oxides, protected amino, protected carboxy, or protected hydroxamic acid derivatives thereof.

2. A compound of formula (I) as defined in claim 1, independent of use, wherein Y is OH, and B-N-B is optionally substituted heterocycloalkyl and/or CR¹R² is optionally substituted cycloalkyl or heterocycloalkyl.

3. A compound of formula (I) as defined in claim 1, independent of use, wherein Y is NHOH.

4. A compound of any preceding claim, wherein X is SO₂

5. A compound of any preceding claim, wherein R³ is C₁₋₆ alkyl, C₂₋₆ alkenyl-R⁵, halogen, CN, NO₂, N(R⁴)₂, OR⁴, COR⁴, NR⁴R⁵, S(O)₀₋₂R⁶ or SO₂N(R⁴)₂.

6. A compound of any preceding claim, wherein R⁷ is CON(R⁴)₂, NR⁴R⁵, SO₂N(R⁴)₂ or cycloimidyl.

7. A compound of any preceding claim, wherein R¹ is optionally substituted C₁₋₆ alkyl, C₁₋₆ alkyl-heteroaryl, or C₁₋₆ alkyl-heterocycloalkyl.

8. A compound of any of claims 1 to 6, wherein CR¹R² is optionally substituted cycloalkyl or heterocycloalkyl.

9. A compound of any preceding claim, wherein independently each B is optionally substituted alkyl, cycloalkyl, heterocycloalkyl, C₁₋₆ alkyl-aryl or C₁₋₆ alkyl-heteroaryl.

10. A compound of any of claims 1 to 9, wherein B-N-B is optionally substituted heterocycloalkyl optionally substituted with =NOR⁴.

11. A compound of any of claims 1 to 3, selected from
methyl 2-((2-phenoxyethylsulfamoyl)methyl)-5-(3,4,4-trimethyl-2,5-dioxoimidazolidin-1-yl)pentanoate,
methyl 2-((N-methyl-(2-phenoxyethyl)sulfamoyl)methyl)-5-(3,4,4-trimethyl-2,5-dioxoimidazolidin-1-yl)pentanoate,
methyl 2-((N-methyl-(2-(4-chlorophenoxy)ethyl)sulfamoyl)methyl)-5-(3,4,4-trimethyl-2,5-dioxoimidazolidin-1-yl)pentanoate,
*tert*-butyl 2-((N-methyl-(benzofuran-2-yl)methylsulfamoyl)methyl)-5-(3,4,4-trimethyl-2,5-dioxoimidazolidin-1-yl)pentanoate,
2-((2-phenoxyethylsulfamoyl)methyl)-5-(3,4,4-trimethyl-2,5-dioxoimidazolidin-1-yl)pentanoic acid,
2-((N-methyl-(2-phenoxyethyl)sulfamoyl)methyl)-5-(3,4,4-trimethyl-2,5-dioxoimidazolidin-1-yl)pentanoic acid,
2-((N-methyl-(2-(4-chlorophenoxy)ethyl)sulfamoyl)methyl)-5-(3,4,4-trimethyl-2,5-dioxoimidazolidin-1-yl)pentanoic acid,
2-((N-methyl-(benzofuran-2-yl)methylsulfamoyl)methyl)-5-(3,4,4-trimethyl-2,5-dioxoimidazolidin-1-yl)pentanoic acid,
2-((2-phenoxyethylsulfamoyl)methyl)-5-(3,4,4-trimethyl-2,5-dioxoimidazolidin-1-yl)pentanoic add N-hydroxy amide,
2-((N-methyl-(2-phenoxyethyl)sulfamoyl)methyl)-5-(3,4,4-trimethyl-2,5-dioxoimidazolidin-1-yl)pentanoic acid N-hydroxy amide,
2-((N-methyl-(2-(4-chlorophenoxy)ethyl)sulfamoyl)methyl)-5-(3,4,4-trimethyl-2,5-dioxoimidazolidin-1-yl)pentanoic acid N-hydroxy amide, and
2-((N-methyl-(benzofuran-2-yl)methyl-sulfamoyl)methyl)-5-(3,4,4-trimethyl-2,5-dioxoimidazolidin-1-yl)pentanoic acid N-hydroxy amide.

12. A compound of any of claims 1 to 5, selected from
ethyl 4-(4-(4-chlorophenyl)piperazin-1-yl)sulfonylmethyl)-tetrahydropyran-4-carboxylate,
ethyl 4-(4-pyridyloxypiperidin-1-yl)sulfonylmethyl-tetrahydropyran-4-carboxylate,
ethyl 4-(4-(4-cyanophenoxy)piperidin-1-yl)sulfonylmethyl-tetrahydropyran-4-carboxylate,
ethyl 4-(*N*-(2-(4-chlorophenoxy)ethyl)-*N*-methylsulfamoyl-methyl)tetrahydropyran-4-carboxylate,
ethyl 4-(4-(4-chlorophenoxy)piperidin-1-yl)sulfonylmethyl-tetrahydropyran-4-carboxylate,
*tert*-butyl2-(*N*-(2-(4-chlorophenoxy)ethyl)-*N*-methyl-sulfamoylmethyl)-3-methylbutanoate,
*tert*-butyl 2-[*N*-methyl-*N*-(2-(4-chlorophenoxy)-2,2-dimethylethyl)sulfamoyl]-methyl-3-methylbutanoate,
*tert*-butyl 2-(4-(4-cyanophenoxyimino)piperidine-1-sulfonylmethyl)-5-(3,4,4-trimethyl-2,5-dioxoimidazolidin-1-yl)pentanoate,
*tert*-butyl 2-(4-(4-cyanophenoxyimino)piperidin-1-ylsulfonylmethyl)-3-methylbutanoic acid,
*tert*-butyl 2-(4-(4-chlorobenzyloxyimino)piperidin-1-ylsulfonylmethyl)-3-methylbutanoate,
4-(4-(4-chlorophenyl)piperazin-1-yl)sulfonylmethyl)-tetrahydropyran-4-carboxylic acid,
4-(4-pyridyloxypiperidin-1-yl)sulfonylmethyl)-tetrahydropyran-4-carboxylic acid,
4-(4-(4-cyanophenoxy)piperidin-1-yl)sulfonylmethyl-tetrahydropyran-4-carboxylic acid,
4-(*N*-(2-(4-chlorophenoxy)ethyl)-*N*-methylsulfamoyl-methyl)tetrahydropyran-4-carboxylic acid,
4-(4-(4-chlorophenoxy)piperidin-1-yl)sulfonylmethyl-tetrahydropyran-4-carboxylic acid,
2-(*N*-(2-(4-chlorophenoxy)ethyl)-*N*-methylsulfamoyl-methyl)-3-methylbutanoic acid,
2-(N-methyl-N-(2-(4-chlorophenoxy)-2,2-dimethylethyl)-sulfamoylmethyl)-3-methylbutanoic acid,
2-(4-(4-cyanophenoxyimino)piperidin-1-ylsulfonylmethyl)-5-(3,4,4-trimethyl-2,5-dioxo-imidazolidin-1-yl)pentanoic acid,
2-(4-(4-cyanophenoxyimino)piperidin-1-ylsulfonylmethyl)-3-methylbutanoic acid,
2-(4-(4-chlorobenzyloxyimino)piperidin-1-ylsulfonylmethyl)-3-methylbutanoic acid,
2-(N-methyl-N-(2-(4-chlorophenoxy)ethyl)-sulfamoylmethyl)-3-methylbutanoic acid N-hydroxy amide,
2-(N-methyl-N-(2-(4-chlorophenoxy)-2,2-dimethyl-ethyl)sulfamoylmethyl)-3-methylbutanoic acid N-hydroxy amide,
4-(4-(4-chlorophenyl)piperazin-1-yl)sulfonylmethyl)-tetrahydropyran-4-carboxylic acid *N*-hydroxy amide hydrochloride,
4-(4-(4-pyridyloxy)piperidin-1-yl)sulfonylmethyl)-tetrahydropyran-4-carboxylic acid *N*-hydroxy amide hydrochloride,
4-(4-(4-cyanophenoxy)piperidin-1-yl)sulfonylmethyl-tetrahydropyran-4-carboxylic acid *N*-hydroxy amide,
4-(*N*-(2-(4-chlorophenoxy)ethyl)-*N*-methylsulfamoyl-methyl)tetrahydropyran-4-carboxylic acid *N*-hydroxy amide,
4-(4-(4-chlorophenoxy)piperidin-1-yl)sulfonylmethyl-tetrahydropyran-4-carboxylic acid *N*-hydroxy amide,
2-[4-(4-cyanophenoxyimino)piperidin-1-ylsulfonylmethyl]-5-(3,4,4-trimethyl-2,5-dioxoimidazolidin-1-yl)pentanoic acid N-hydroxy amide,
2-(4-(4-cyanophenoxyimino)piperidin-1-ylsulfonylmethyl)-3-methylbutanoic acid N-hydroxy amide,
2-(4-(4-chlorobenzyloxyimino)piperidin1-ylsulfonylmethyl)-3-methylbutanoic acid N-hydroxy amide,
2-((N-ethyl-N-(2-methoxyethyl)-sulfamoylmethyl)-5-(3,4,4-trimethyl-2,5-dioxoimidazolidin-1-yl)-pentanoic acid N-hydroxy amide,
2-(3,3-diphenylpropylsulfamoylmethyl)-5-(3,4,4-trimethyl-2,5-dioxo-imidazolidin-1-yl)-pentanoic acid N-hydroxy amide,
2-(cyclohexylmethylsulfamoylmethyl)-5-(3,4,4-trimethyl-2,5-dioxoimidazolidin-1-yl)-pentanoic acid N-hydroxy amide,
2-(1-methyl-3-phenylpropylsulfamoylmethyl)-5-(3,4,4-trimethyl-2,5-dioxoimidazolidin-1-yl)pentanoic acid N-hydroxy amide,
2-((3-phenylpropyl)sulfamoylmethyl)-5-(3,4,4-trimethyl-2,5-dioxo-imidazolidin-1-yl)-pentanoic acid N-hydroxy amide,
2-((N-methyl-N-octylsulfamoyl)methyl)-5-(3,4,4-trimethyl-2,5-dioxoimidazolidin-1-yl)-pentanoic acid N-hydroxy amide,
2-(N-(2-cyanoethyl)-N-octylsulfamoylmethyl)-5-(3,4,4-trimethyl-2,5-dioxoimidazolidin-1-yl)-pentanoic acid N-hydroxy amide,
2-(N-(2-(4-chlorophenoxy)-1-methylethyl)-N-methylsulfamoylmethyl)-5-(3,4,4-trimethyl-2,5-dioxo-imidazolidin-1-yl)-pentanoic acid N-hydroxy amide,
2-(N-methyl-N-phenethylsulfamoylmethyl)-5-(3,4,4-trimethyl-2,5-dioxoimidazolidin-1-yl)-pentanoic acid N-hydroxy amide,
2-(N-(2-(2-chlorophenoxy)ethyl)-N-methyl-sulfamoylmethyl)-5-(3,4,4-trimethyl-2,5-dioxoimidazolidin-1-yl)-pentanoic acid N-hydroxy amide,
2-(N-ethyl-N-phenylsulfamoylmethyl)-5-(3,4,4-trimethyl-2,5-dioxo-imidazolidin-1-yl)-pentanoic acid N-hydroxy amide and
2-(N-(4-chlorophenyl)-N-methylsulfamoylmethyl)-5-(3,4,4-trimethyl-2,5-dioxoimidazolidin-1-yl)pentanoic acid N-hydroxy amide.

13. A compound of any preceding claim, in the form of a single enantiomer or diastereomer.

14. A pharmaceutical composition for use in therapy, comprising a compound of any preceding claim, and a pharmaceutically-acceptable diluent or carrier.

15. Use of a compound of any of claims 1 to 13, for the manufacture of a medicament for the treatment or prevention of a condition associated with matrix metalloproteinases or that is mediated by TNF α or enzymes involved in the shedding of *L*-selectin, CD23, the TNF receptors, IL-6 receptors or IL-1 receptors.

16. Use according to claim 15, wherein the condition is selected from cancer, inflammation and inflammatory diseases, tissue degeneration, periodontal disease, ophthalmological disease, dermatological disorders, fever, cardiovascular effects, haemorrhage, coagulation and acute phase response, cachexia, anorexia, acute infection, HIV infection, shock states, graft versus host reactions, autoimmune disease, reperfusion injury, meningitis, migraine and aspirin-independent anti-thrombosis.

17. Use according to claim 15, wherein the condition is selected from tumour growth, angiogenesis, tumour invasion and spread, metastases, malignant ascites and malignant pleural effusion.

18. Use according to claim 15, wherein the condition is selected from cerebral ischaemia, ischaemic heart disease, rheumatoid arthritis, osteoarthritis, osteoporosis, asthma, multiple sclerosis, neurodegeneration, Alzheimer's, atherosclerosis, stroke, vasculitis, Crohn's disease and ulcerative colitis.

19. Use according to claim 15, wherein the condition is selected from corneal ulceration, retinopathy and surgical wound healing.

20. Use according to claim 15, wherein the condition is selected from psoriasis, atopic dermatitis, chronic ulcers and epidermolysis bullosa.

21. Use according to claim 15, wherein the condition is selected from periodontitis and gingivitis.

22. Use according to claim 15, wherein the condition is selected from rhinitis, allergic conjunctivitis, eczema and anaphylaxis.

23. Use according to claim 15, wherein the condition is selected from restenosis, congestive heart failure, endometriosis, atherosclerosis and endosclerosis.

24. Use according to claim 15, wherein the condition is selected from pelvic inflammatory disease (PID), age-related macular degeneration and cancer-induced bone resorption.

25. Use according to claim 15, wherein the condition is a lung disease.

26. Use according to claim 25, wherein the condition is selected from cystic fibrosis adult respiratory distress syndrome (ARDS), emphysema, bronchitis obliterans-organising pneumonia (BOOP), idiopathic pulmonary fibrosis (PIP), diffuse alveolar damage, pulmonary Langerhan's cell granulamatosis, pulmonarylymphangioleiomyomatosis (LAM) and chronic obstructive pulmonary disease (COPD).

## Patentansprüche

1. Verbindung für die therapeutische Verwendung dargestellt durch Formel (I): 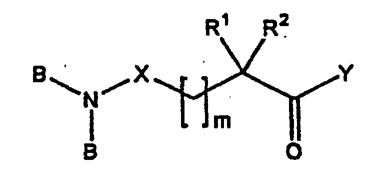 worin
m 0-2 ist,
X S(O)₁₋₂ ist,
Y OH oder NHOH ist,
R¹ H oder eine Gruppe (gegebenenfalls substituiert mit R⁷) ausgewählt aus C₁₋₆-Alkyl, C₂₋₆-Alkenyl, Aryl, C₁₋₆-Alkylaryl, Heteroaryl, C₁₋₆-Alkylheteroaryl, Heterocycloalkyl, C₁₋₆-Alkylheterocycloalkyl, Cycloalkyl und C₁₋₆-Alkylcycloalkyl ist und R² H oder C₁₋₆-Alkyl ist,
oder CR¹R² ein Cycloalkyl- oder Heterocycloalkylring ist, gegebenenfalls substituiert mit R⁷ oder einer Gruppe (gegebenenfalls substituiert mit R⁷) ausgewählt aus C₁₋₆-Alkyl, Aryl, C₁₋₆-Alkylaryl, Heteroaryl und C₁₋₆-Alkylheteroaryl,
jedes B gleich oder verschieden ist und H oder eine Gruppe ausgewählt aus C₁₋₆-Alkylaryl, C₁₋₆-Alkyl, Cycloalkyl, C₁₋₆-Alkylcycloalkyl, Cycloalkenyl, Heterocycloalkenyl, C₁₋₆-Alkylheteroaryl, Heterocycloalkyl, C₁₋₆-Alkylheterocycloalkyl, Aryl oder Heteroaryl ist, wobei jede dieser Gruppen gegebenenfalls substituiert ist durch einen Substituenten ausgewählt aus R³, C₁₋₆-Alkyl-R³, C₂₋₆-Alkenyl-R³, Aryl (gegebenenfalls substituiert mit R³), Aryl-C₁₋₆-alkyl-R³, C₁₋₆-Alkylaryl (gegebenenfalls substituiert mit R³), C₁₋₆-Alkylheteroaryl (gegebenenfalls substituiert mit R³), Aryl-C₂₋₆-alkenyl-R⁵, Heteroaryl (gegebenenfalls substituiert mit R³), Heteroaryl-C₁₋₆-alkyl-R³, Cycloalkyl (gegebenenfalls substituiert mit R³) und Heterocycloalkyl (gegebenenfalls substituiert mit R³), mit der Maßgabe, dass NB₂ nicht NH₂ ist,
oder B-N-B ein Heterocycloalkylring ist, der mit =O oder =NOR⁴ substituiert ist,
oder, wenn weder R¹ noch R² H sind, B-N-B ein Heterocycloalkyl- oder Heterocycloalkenylring ist, der gegebenenfalls substituiert ist durch einen Substituenten ausgewählt aus R³, C₁₋₆-Alkyl-R³, C₂₋₆-Alkenyl-R³, Aryl (gegebenenfalls substituiert mit R³), Aryl-C₁₋₆-alkyl-R³, C₁₋₆-Alkylaryl (gegebenenfalls substituiert mit R³), C₁₋₆-Alkylheteroaryl (gegebenenfalls substituiert mit R³), Aryl-C₂₋₆-alkenyl-R⁵, Heteroaryl (gegebenenfalls substituiert mit R³), Heteroaryl-C₁₋₆-alkyl-R³, Cycloalkyl (gegebenenfalls substituiert mit R³) und Heterocycloalkyl (gegebenenfalls substituiert mit R³),
R³ C₁₋₆-Alkyl, C₂₋₆-Alkenyl-R⁵, Halogen, CN, NO₂, N(R⁴)₂, OR⁴, C(=NOR⁶)R⁴, CON(R⁴)₂, COR⁴, CO₂R⁸, NR⁴R⁵, S(O)₀₋₂R⁶ oder SO₂N(R⁴)₂ ist,
R⁴ H oder eine Gruppe ausgewählt aus C₁₋₆-Alkyl, Aryl, C₁₋₆-Alkylaryl, Heteroaryl, C₁₋₆-Alkylheteroaryl, Cycloalkyl, C₁₋₆-Alkylcycloalkyl, Heterocycloalkyl und C₁₋₆-Alkylheterocycloalkyl ist, wobei die genannte Gruppe gegebenenfalls substituiert ist mit R⁶, COR⁶, SO₀₋₂R⁶, CO₂R⁶, OR⁶, CONR⁸R⁶, NR⁸R⁶, Halogen, CN, SO₂NR⁸R⁶ oder NO₂ und für jeden Fall von N(R⁴)₂ die R⁴-Gruppen gleich oder verschieden sind oder N(R⁴)₂ Heterocycloalkyl ist, gegebenenfalls substituiert mit R⁶, COR⁶, SO₀₋₂R⁶, CO₂R⁶, OR⁶, CONR⁸R⁶, NR⁸R⁶, Halogen, CN, SO₂NR⁸R⁶ oder NO₂,
R⁵ COR⁴, CON(R⁴)₂, CO₂R⁶ oder SO₂R⁶ ist,
R⁶ C₁₋₆-Alkyl, Aryl, C₁₋₆-Alkylaryl, Heteroaryl oder C₁₋₆-Alkylheteroaryl ist und
R⁷ OR⁴, COR⁴, CO₂R⁸, CON(R⁴)₂, NR⁴R⁵, S(O)₀₋₂R⁶, SO₂N(R⁴)₂, Halogen, CN oder Cycloimidyl (gegebenenfalls substituiert mit R⁸) ist und
R⁸ H oder C₁₋₆-Alkyl ist,
und deren Salze, Solvate, Hydrate, N-Oxide, geschützte Amino-, geschützte Carboxy- oder geschützte Hydroxamsäure-Derivate.

2. Verbindung nach Formel (I) wie in Anspruch 1 definiert, unabhängig von der Verwendung, worin Y OH ist und B-N-B gegebenenfalls substituiertes Heterocycloalkyl ist und/oder CR¹R² gegebenenfalls substituiertes Cycloalkyl oder Heterocycloalkyl ist.

3. Verbindung nach Formel (I) wie in Anspruch 1 definiert, unabhängig von der Verwendung, worin Y NHOH ist.

4. Verbindung nach irgendeinem vorhergehenden Anspruch, worin X SO₂ ist.

5. Verbindung nach irgendeinem vorhergehenden Anspruch, worin R³ C₁₋₆-Alkyl, C₂₋₆-Alkenyl-R⁵, Halogen, CN, NO₂, N(R⁴)₂, OR⁴, COR⁴, NR⁴R⁵, S(O)₀₋₂R⁶ oder SO₂N(R⁴)₂ ist.

6. Verbindung nach irgendeinem vorhergehenden Anspruch, worin R⁷ CON(R⁴)₂, NR⁴R⁵, SO₂N(R⁴)₂ oder Cycloimidyl ist.

7. Verbindung nach irgendeinem vorhergehenden Anspruch, worin R¹ gegebenenfalls substituiertes C₁₋₆-Alkyl, C₁₋₆-Alkylheteroaryl oder C₁₋₆-Alkylheterocycloalkyl ist.

8. Verbindung nach irgendeinem der Ansprüche 1 bis 6, worin CR¹R² gegebenenfalls substituiertes Cycloalkyl oder Heterocycloalkyl ist.

9. Verbindung nach irgendeinem vorhergehenden Anspruch, worin jedes B unabhängig gegebenenfalls substituiertes Alkyl, Cycloalkyl, Heterocycloalkyl, C₁₋₆-Alkylaryl oder C₁₋₆-Alkylheteroaryl ist.

10. Verbindung nach irgendeinem der Ansprüche 1 bis 9, worin B-N-B gegebenenfalls substituiertes Heterocycloalkyl, gegebenenfalls substituiert mit =NOR⁴ ist.

11. Verbindung nach irgendeinem der Ansprüche 1 bis 3 ausgewählt aus
Methyl-2-((2-phenoxyethylsulfamoyl)methyl)-5-(3,4,4-trimethyl-2,5-dioxoimidazolidin-1-yl)pentanoat,
Methyl-2-((N-methyl(2-phenoxyethyl)sulfamoyl)methyl)-5-(3,4,4-trimethyl-2,5-dioxoimidazolidin-1-yl)pentanoat,
Methyl-2-((N-methyl-(2-(4-chlorphenoxy)ethyl)sulfamoyl)methyl)-5-(3,4,4-trimethyl-2,5-dioxoimidazolidin-1-yl)pentanoat,
tert.-Butyl-2-((N-methyl-(benzofuran-2-yl)methylsulfamoyl)methyl)-5-(3,4,4-trimethyl-2,5-dioxoimidazolidin-1-yl)pentanoat,
2-((2-Phenoxyethylsulfamoyl)methyl)-5-(3,4,4-trimethyl-2,5-dioxoimidazolidin-1-yl)pentansäure,
2-((N-Methyl-(2-phenoxyethyl)sulfamoyl)methyl)-5-(3,4,4-trimethyl-2,5-dioxoimidazolidin-1-yl)pentansäure,
2-((N-Methyl-(2-(4-chlorphenoxy)ethyl)sulfamoyl)methyl)-5-(3,4,4-trimethyl-2,5-dioxoimidazolidin-1-yl)pentansäure,
2-((N-Methyl-(benzofuran-2-yl)methylsulfamoyl)methyl)-5-(3,4,4-trimethyl-2,5-dioxoimidazolidin-1-yl)pentansäure,
2-((2-Phenoxyethylsulfamoyl)methyl)-5-(3,4,4-trimethyl-2,5-dioxoimidazolidin-1-yl)pentansäure-N-hydroxyamid,
2-((N-Methyl-(2-phenoxyethyl)sulfamoyl)methyl)-5-(3,4,4-trimethyl-2,5-dioxoimidazolidin-1-yl)pentansäure-N-hydroxyamid,
2-((N-Methyl(2-(4-chlorphenoxy)ethyl)sulfamoyl)methyl)-5-(3,4,4-trimethyl-2,5-dioxoimidazolidin-1-yl)pentansäure-N-hydroxyamid und
2-((N-Methyl(benzofuran-2-yl)methylsulfamoyl)methyl)-5-(3,4,4-trimethyl-2,5-dioxoimidazolidin-1-yl)pentansäure-N-hydroxyamid.

12. Verbindung nach irgendeinem der Ansprüche 1 bis 5 ausgewählt aus
Ethyl-4-(4-(4-chlorphenyl)piperazin-1-yl)sulfonylmethyl)tetrahydropyran-4-carboxylat,
Ethyl-4-(4-pyridyloxypiperidin-1-yl)sulfonylmethyltetrahydropyran-4-carboxylat,
Ethyl-4-(4-(4-cyanophenoxy)piperidin-1-yl)sulfonylmethyltetrahydropyran-4-carboxylat,
Ethyl-4-(N-(2-(4-chlorphenoxy)ethyl)-N-methylsulfamoylmethyl)tetrahydropyran-4-carboxylat,
Ethyl-4-(4-(4-chlorphenoxy)piperidin-1-yl)sulfonylmethyltetrahydropyran-4-carboxylat,
tert.-Butyl-2-(N-(2-(4-chlorphenoxy)ethyl)-N-methylsulfamoylmethyl)-3-methylbutanoat,
tert.-Butyl-2-[N-methyl-N-(2-(4-chlorphenoxy)-2,2-dimethylethyl)sulfamoyl]methyl-3-methylbutanoat,
tert.-Butyl-2-(4-(4-cyanophenoxyimino)piperidin-1-sulfonylmethyl)-5-(3,4,4-trimethyl-2,5-dioxoimidazolidin-1-yl)pentanoat,
tert.-Butyl-2-(4-(4-cyanophenoxyimino)piperidin-1-ylsulfonylmethyl)-3-methylbutansäure,
tert.-Butyl-2-(4-(4-chlorbenzyloxyimino)piperidin-1-ylsulfonylmethyl)-3-methylbutanoat,
4-(4-(4-Chlorphenyl)piperazin-1-yl)sulfonylmethyl)tetrahydropyran-4-carbonsäure,
4-(4-Pyridyloxypiperidin-1-yl)sulfonylmethyl)tetrahydropyran-4-carbonsäure,
4-(4-(4-Cyanophenoxy)piperidin-1-yl)sulfonylmethyltetrahydropyran-4-carbonsäure,
4-(N-(2-(4-Chlorphenoxy)ethyl)-N-methylsulfamoylmethyl)tetrahydropyran-4-carbonsäure,
4-(4-(4-Chlorphenoxy)piperidin-1-yl)sulfonylmethyltetrahydropyran-4-carbonsäure,
2-(N-(2-(4-Chlorphenoxy)ethyl)-N-methylsulfamoylmethyl)-3-methylbutansäure,
2-(N-Methyl-N-(2-(4-chlorphenoxy)-2,2-dimethylethyl)sulfamoylmethyl)-3-methylbutansäure,
2-(4-(4-Cyanophenoxyimino)piperidin-1-ylsulfonylmethyl)-5-(3,4,4-trimethyl-2,5-dioxoimidazolidin-1-yl)pentansäure,
2-(4-(4-Cyanophenoxyimino)piperidin-1-ylsulfonylmethyl)-3-methylbutansäure,
2-(4-(4-Chlorbenzyloxyimino)piperidin-1-ylsulfonylmethyl)-3-methylbutansäure,
2-(N-Methyl-N-(2-(4-chlorphenoxy)ethyl)sulfamoylmethyl)-3-methylbutansäure-N-hydroxyamid,
2-(N-Methyl-N-(2-(4-chlorphenoxy)-2,2-dimethylethyl)sulfamoylmethyl)-3-methylbutansäure-N-hydroxyamid,
4-(4-(4-Chlorphenyl)piperazin-1-yl)sulfonylmethyl)tetrahydropyran-4-carbonsäure-N-hydroxyamidhydrochlorid,
4-(4-(4-Pyridyloxy)piperidin-1-yl)sulfonylmethyl)tetrahydropyran-4-carbonsäure-N-hydroxyamidhydrochlorid,
4-(4-(4-Cyanophenoxy)piperidin-1-yl)sulfonylmethyltetrahydropyran-4-carbonsäure-N-hydroxyamid,
4-(N-(2-(4-Chlorphenoxy)ethyl)-N-methylsulfamoylmethyl)tetrahydropyran-4-carbonsäure-N-hydroxyamid,
4-(4-(4-Chlorphenoxy)piperidin-1-yl)sulfonylmethyltetrahydropoyran-4-carbonsäure-N-hydroxyamid,
2-[4-(4-Cyanophenoxyimino)piperidin-1-ylsulfonytmethyl]-5-(3,4,4-trimethyl-2,5-dioxoimidazolidin-1-yl)pentansäure-N-hydroxyamid,
2-(4-(4-Cyanophenoxyimino)piperidin-1-ylsulfonylmethyl)-3-methylbutansäure-N-hydroxyamid,
2-(4-(4-Chlorbenzyloxyimino)piperidin-1-ylsulfonylmethyl)-3-methylbutansäure-N-hydroxyamid,
2-((N-Ethyl-N-(2-methoxyethyl)sulfamoylmethyl)-5-(3,4,4-trimethyl-2,5-dioxoimidazolidin-1-yl)pentansäure-N-hydroxyamid,
2-(3,3-Diphenylpropylsulfamoylmethyl)-5-(3,4,4-trimethyl-2,5-dioxoimidazolidin-1-yl)pentansäure-N-hydroxyamid,
2-(Cyclohexylmethylsulfamoylmethyl)-5-(3,4,4-trimethyl-2,5-dioxoimidazolidin-1-yl)pentansäure-N-hydroxyamid,
2-(1-Methyl-3-phenylpropylsulfamoylmethyl)-5-(3,4,4-trimethyl-2,5-dioxoimidazolidin-1-yl)pentansäure-N-hydroxyamid,
2-((3-Phenylpropyl)sulfamoylmethyl)-5-(3,4,4-trimethyl-2,5-dioxoimidazolidin-1-yl)pentansäure-N-hydroxyamid,
2-((N-Methyl-N-octylsulfamoyl)methyl)-5-(3,4,4-trimethyl-2,5-dioxoimidazolidin-1-yl)pentansäure-N-hydroxyamid,
2-(N-(2-Cyanoethyl)-N-octylsulfamoylmethyl)-5-(3,4,4-trimethyl-2,5-dioxoimidazolidin-1-yl)pentansäure-N-hydroxyamid,
2-(N-(2-(4-Chlorphenoxy)-1-methylethyl)-N-methylsulfamoylmethyl)-5-(3,4,4-trimethyl-2,5-dioxoimidazolidin-1-yl)pentansäure-N-hydroxyamid,
2-(N-Methyl-N-phenethylsulfamoylmethyl)-5-(3,4,4-trimethyl-2,5-dioxoimidazolidin-1-yl)pentansäure-N-hydroxyamid,
2-(N-(2-(2-Chlorphenoxy)ethyl)-N-methylsulfamoylmethyl)-5-(3,4,4-trimethyl-2,5-dioxoimidazolidin-1-yl)pentansäure-N-hydroxyamid,
2-(N-Ethyl-N-phenylsulfamoylmethyl)-5-(3,4,4-trimethyl-2,5-dioxoimidazolidin-1-yl)pentansäure-N-hydroxyamid und
2-(N-(4-Chlorphenyl)-N-methylsulfamoylmethyl)-5-(3,4,4-trimethyl-2,5-dioxoimidazolidin-1-yl)pentansäure-N-hydroxyamid.

13. Verbindung nach irgendeinem vorhergehenden Anspruch in Form eines Enantiomers oder Diastereomers.

14. Pharmazeutische Zusammensetzung zur Verwendung bei der Therapie umfassend eine Verbindung nach irgendeinem vorhergehenden Anspruch und ein pharmazeutisch verträgliches Verdünnungsmittel oder einen pharmazeutisch verträglichen Träger.

15. Verwendung einer Verbindung nach irgendeinem der Ansprüche 1 bis 13 zur Herstellung eines Arzneimittels zur Behandlung oder Verhinderung eines Zustands, der mit Matrix-Metalloproteinasen assoziiert ist oder der durch TNFα oder Enzyme, die bei der Freisetzung (Shedding) von L-Selectin, CD23, den TNF-Rezeptoren, IL-6-Rezeptoren oder IL-1-Rezeptoren beteiligt sind, vermittelt wird.

16. Verwendung nach Anspruch 15, worin der Zustand ausgewählt ist aus Krebs, Entzündung und Entzündungskrankheiten, Gewebedegeneration, Wurzelhautentzündung, Augenkrankheiten, dermatologischen Störungen, Fieber, kardiovaskulären Effekten, Hämorrhagie, Gerinnung und Akutphasen-Reaktion, Kachexie, Anorexie, akuter Infektion, HIV-Infektion, Schockzuständen, Transplantat-Wirt-Reaktionen, Autoimmunkrankheiten, Reperfusionsschädi-gung, Meningitis, Migräne und Aspirin-unabhängiger Thromboseprophylaxe.

17. Verwendung nach Anspruch 15, worin der Zustand ausgewählt ist aus Tumorwachstum, Angiogenese, Tumorinvasion und -ausbreitung, Metastasen, malignem Ascites und malignem Pleuraerguss.

18. Verwendung nach Anspruch 15, worin der Zustand ausgewählt ist aus zerebraler Ischämie, ischämischer Herzerkrankung, rheumatoider Arthritis, Osteoarthritis, Osteoporose, Asthma, Multipler Sklerose, Neurodegeneration, Alzheimer-Krankheit, Arteriosklerose, Schlaganfall, Vaskulitis, Morbus Crohn und ulzeröser Kolitis.

19. Verwendung nach Anspruch 15, worin der Zustand ausgewählt ist aus Hornhautgeschwüren, Retinopathie und Heilung einer Operationswunde.

20. Verwendung nach Anspruch 15, worin der Zustand ausgewählt ist aus Psoriasis, atopischer Dermatitis, chronischen Geschwüren und Epidermolysis bultosa.

21. Verwendung nach Anspruch 15, worin der Zustand ausgewählt ist aus Periodontitis und Gingivitis.

22. Verwendung nach Anspruch 15, worin der Zustand ausgewählt ist aus Rhinitis, allergischer Konjunktivitis, Exzem und Anaphylaxie.

23. Verwendung nach Anspruch 15, worin der Zustand ausgewählt ist aus Restenose, Stauungsherzinsuffizienz, Endometriose, Arteriosklerose und Endosklerose.

24. Verwendung nach Anspruch 15, worin der Zustand ausgewählt ist aus Salpingitis (PID, "pelvic inflammatory disease"), altersbedingter Makuladegeneration und durch Krebs-induzierter Knochenresorption.

25. Verwendung nach Anspruch 15, worin der Zustand eine Lungenkrankheit ist.

26. Verwendung nach Anspruch 25, worin der Zustand ausgewählt ist aus zystischer Fibrose, Atemnotsyndrom des Erwachsenen (ARDS, "adult respiratory distress syndrome"), Emphysem, Bronchiolitis obliteransorganisierender Pneumonie (BOOP), idiopathischer Lungenfibrose (PIF, "idiopathic pulmonary fibrosis"), diffusem Alveolarschaden, Langerhanszell-Lungengranulomatose, Lungen-Lymphangioleiomyomatose (LAM) und chronisch-obstruktiver Lungenerkrankung (COLD).

## Revendications

1. Composé destiné à une utilisation thérapeutique, représenté par la formule (I) : 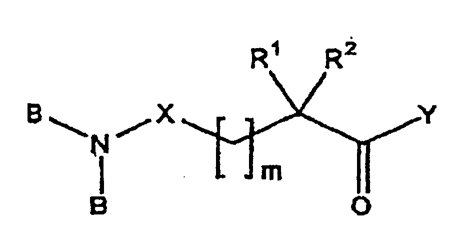 dans laquelle
m vaut de 0 à 2 ;
X est S(O)₁₋₂,
Y est OH ou NHOH ;
R¹ est H ou un groupe (éventuellement substitué par R⁷) choisi parmi un alkyle en C₁₋₆, un alcényle en C₂₋₆, un aryle, un C₁₋₆-alkylaryle, un hétéroaryle, un C₁₋₆-alkylhétéroaryle, un hétérocycloalkyle, un C₁₋₆-alkylhétérocycloalkyle, un cycloalkyle et un C₁₋₆-alkylcycloalkyle, et R² est H ou un alkyle en C₁₋₆ ;
ou CR¹R² est un noyau cycloalkyle ou hétérocycloalkyle éventuellement substitué par R⁷ ou un groupe (éventuellement substitué par R⁷) choisi parmi un alkyle en C₁₋₆, un aryle, un C₁₋₆-alkylaryle, un hétéroaryle et un C₁₋₆-alkylhétéroaryle ;
chaque B est identique ou différent et est H ou un groupe choisi parmi un C₁₋₆-alkylaryle, un alkyle en C₁₋₆, un cycloalkyle, un C₁₋₆-alkylcycloalkyle, un cycloalcényle, un hétérocycloalcényle, un C₁₋₆-alkylhétéroaryle, un hétérocycloalkyle, un C₁₋₆-alkylhétérocycloalkyle, un aryle ou un hétéroaryle, l'un quelconque de ces groupes étant éventuellement substitué par un substituant choisi parmi R³, un C₁₋₆-alkyl-R³, un C₂₋₆-alcényl-R³, un aryle (éventuellement substitué par R³), un aryl-C₁₋₆-alkyl-R³, un C₁₋₆-alkylaryle (éventuellement substitué par R³), un C₁₋₆-alkylhétéroaryle (éventuellement substitué par R³), un aryl-C₂₋₆-alcényl-R⁵, un hétéroaryle (éventuellement substitué par R³), un hétéroaryl-C₁₋₆-alkyl-R³, un cycloalkyle (éventuellement substitué par R³) et un hétérocycloalkyle (éventuellement substitué par R³), à condition que NB₂ ne soit pas NH₂,
ou B-N-B est un noyau hétérocycloalkyle substitué par =O ou =NOR⁴,
ou lorsque ni R¹ ni R² est H, B-N-B est un noyau hétérocycloalkyle ou hétérocycloalcényle éventuellement substitué par un substituant choisi parmi R³, un C₁₋₆-alkyl-R³, un C₂₋₆-alcényl-R³, un aryle (éventuellement substitué par R³), un aryl-C₁₋₆-alkyl-R³, un C₁₋₆-alkylaryle (éventuellement substitué par R³), un C₁₋₆-alkylhétéroaryle (éventuellement substitué par R³), un aryl-C₂₋₆-alcényl-R⁵, un hétéroaryle (éventuellement substitué par R³), un hétéroaryl-C₁₋₆-alkyl-R³, un cycloalkyle (éventuellement substitué par R³) et un hétérocycloalkyle (éventuellement substitué par R³) ;
R³ est un alkyle en C₁₋₆ ; un C₂₋₆-alcényl-R⁵, un halogène, CN, NO₂, N(R⁴)₂, OR⁴, C(=NOR⁶)R⁴, CON(R⁴)₂, COR⁴, CO₂R⁸, NR⁴R⁵, S(O)₀₋₂R⁶ ou SO₂N(R⁴)₂ ;
R⁴ est H ou un groupe choisi parmi un alkyle en C₁₋₆, un aryle, un C₁₋₆-alkylaryle, un hétéroaryle, un C₁₋₆-alkylhétéroaryle, un cycloalkyle, un C₁₋₆-alkylcycloalkyle, un hétérocycloalkyle et un C₁₋₆-alkylhétérocycloalkyle, où ledit groupe est éventuellement substitué par R⁶, COR⁶, SO₀₋₂R⁶, CO₂R⁶, OR⁶, CONR⁸R⁶, NR⁸R⁶, un halogène, CN, SO₂NR⁸R⁶ ou NO₂, et pour chaque occurrence de N(R⁴)₂, les groupes R⁴ sont identiques ou différents ou N(R⁴)₂ est un hétérocycloalkyle éventuellement substitué par R⁶, COR⁶, SO₀₋₂R⁶, CO₂R⁶, OR⁶, CONR⁸R⁶, NR⁸R⁶, un halogène, CN, SO₂NR⁸R⁶ ou NO₂ ;
R⁵ est COR⁴, CON(R⁴)₂, CO₂R⁶ ou SO₂R⁶ ;
R⁶ est un alkyle en C₁₋₆, un aryle, un C₁₋₆-alkylaryle, un hétéroaryle ou un C₁₋₆-alkylhétéroaryle ; et
R₇ est OR⁴, COR⁴, CO₂R⁸, CON(R⁴)₂, NR⁴R⁵, S(O)₀₋₂R⁶, SO₂N(R⁴)₂, un halogène, CN ou un cycloimidyle (éventuellement substitué par R⁸) ; et
R⁸ est H ou un alkyle en C₁₋₆ ;
et des sels, solvates, hydrates, N-oxydes, dérivés amino protégés, dérivés carboxy protégés ou dérivés d'acide hydroxamique protégés de celui-ci.

2. Composé de formule (I) telle que définie dans la revendication 1, indépendamment de l'utilisation, dans laquelle Y est OH et B-N-B est un hétérocycloalkyle éventuellement substitué et/ou CR¹R² est un cycloalkyle ou hétérocycloalkyle éventuellement substitué.

3. Composé de formule (I) telle que définie dans la revendication 1, indépendamment de l'utilisation, dans laquelle Y est NHOH.

4. Composé selon l'une quelconque des revendications précédentes, dans lequel X est SO₂.

5. Composé selon l'une quelconque des revendications précédentes, dans lequel R³ est un alkyle en C₁₋₆, un C₂₋₆-alcényl-R⁵, un halogène, CN, NO₂, N(R⁴)₂, OR⁴, COR⁴, NR⁴R⁵, S(O)₀₋₂R⁶ ou SO₂N(R⁴)₂.

6. Composé selon l'une quelconque des revendications précédentes, dans lequel R⁷ est CON(R⁴)₂, NR⁴R⁵, SO₂N(R⁴)₂ ou un cycloimidyle.

7. Composé selon l'une quelconque des revendications précédentes, dans lequel R¹ est un alkyle en C₁₋₆ éventuellement substitué, un C₁₋₆-alkylhétéroaryle ou un C₁₋₆-alkylhétérocycloalkyle.

8. Composé selon l'une quelconque des revendications 1 à 6, dans lequel CR¹R² est un cycloalkyle ou un hétérocycloalkyle éventuellement substitué.

9. Composé selon l'une quelconque des revendications précédentes, dans lequel chaque B est indépendamment un alkyle, un cycloalkyle, un hétérocycloalkyle, un C₁₋₆-alkylaryle ou un C₁₋₆-alkylhétéroaryle, éventuellement substitué.

10. Composé selon l'une quelconque des revendications 1 à 9, dans lequel B-N-B est un hétérocycloalkyle éventuellement substitué par =NOR⁴.

11. Composé selon l'une quelconque des revendications 1 à 3, choisi parmi
le 2((2-phénoxyéthylsulfamoyl)méthyl)-5-(3,4,4-triméthyl-2,5-dioxoimidazolidin-1-yl)pentanoate de méthyle,
le 2-((*N*-méthyl-(2-phénoxyéthyl)sulfamoyl)méthyl)-5-(3,4,4-triméthyl-2,5-dioxoimidazolidin-1-yl)pentanoate de méthyle,
le 2-((*N*-méthyl-(2-(4-chlorophénoxy)éthyl)sulfamoyl)méthyl)-5-(3,4,4-triméthyl-2,5-dioxoimidazolidin-1-yl)pentanoate de méthyle,
le 2-((*N*-méthyl-(benzofuran-2-yl)méthylsulfamoyl)méthyl)-5-(3,4,4-triméthyl-2,5-dioxoimidazolidin-1-yl)pentanoate de *tert*-butyle,
l'acide 2-((2-phénoxyéthylsulfamoyl)méthyl)-5-(3,4,4-triméthyl-2,5-dioxoimidazolidin-1-yl)pentanoïque,
l'acide 2-((*N*-méthyl-(2-phénoxyéthyl)sulfamoyl)méthyl)-5-(3,4,4-triméthyl-2,5-dioxoimidazolidin-1-yl)pentanoïque,
l'acide 2-((*N*-méthyl-(2-(4-chlorophénoxy)éthyl)sulfamoyl)méthyl)-5-(3,4,4-triméthyl-2,5-dioxoimidazolidin-1-yl)pentanoique,
l'acide 2-((*N*-méthyl-(benzofuran-2-yl)méthylsulfamoyl)méthyl)-5-(3,4,4-triméthyl-2,5-dioxoimidazolidin-1-yl)pentanoïque,
le *N*-hydroxy-amide de l'acide 2-((2-phénoxyéthylsulfamoyl)méthyl)-5-(3,4,4-triméthyl-2, 5-dioxoimidazolidin-1-yl)pentanoïque,
le *N*-hydroxy-amide de l'acide 2-((*N*-méthyl-(2-phénoxyéthyl)sulfamoyl)méthyl)-5-(3,4,4-triméthyl-2,5-dioxoimidazolidin-1-yl)pentanoïque,
le *N*-hydroxy-amide de l'acide 2-((*N*-méthyl-(2-(4-chlorophénoxy)éthyl)sulfamoyl)méthyl)-5-(3,4,4-triméthyl-2,5-dioxoimidazolidin-1-yl)pentanoïque et
le *N*-hydroxy-amide de l'acide 2-((*N*-méthyl-(benzofuran-2-yl)méthylsulfamoyl)méthyl)-5-(3,4,4-triméthyl-2,5-dioxoimidazolidin-1-yl)pentanoïque,

12. Composé selon l'une quelconque des revendications 1 à 5, choisi parmi
le 4-(4-(4-chlorophényl)pipérazin-1-yl)sulfonylméthyl)-tétrahydropyrane-4-carboxylate d'éthyle,
le 4-(4-pyridyloxypipéridin-1-yl)sulfonylméthyltétrahydropyrane-4-carboxylate d'éthyle,
le 4-(4-(4-cyanophénoxy)pipéridin-1-yl)sulfonylméthyltétrahydropyrane-4-carboxylate d'éthyle,
le 4-(*N*-(2-(4-chlorophénoxy)éthyl)-*N*-méthylsulfamoylméthyl)tétrahydropyrane-4-carboxylate d'éthyle,
le 4-(4-(4-chlorophénoxy)pipéridin-1-yl)sulfonylméthyltétrahydropyrane-4-carboxylate d'éthyle,
le 2-(*N*-(2-(4-chlorophénoxy)éthyl)-*N*-méthylsulfamoylméthyl)-3-méthylbutanoate de *tert*-butyle,
le 2-[*N*-méthyl-*N*-(2-(4-chlorophénoxy)-2,2-diméthyléthyl)sulfamoyl]méthyl-3-méthylbutanoate de *tert*-butyle,
le 2-(4-(4-cyanophénoxyimino)pipéridine-1-sulfonylméthyl) -5-(3,4,4-triméthyl-2, 5-dioxoimidazolidin-1-yl)pentanoate de *tert*-butyle,
l'acide tert-butyl-2-(4-(4-cyanophénoxyimino)pipéridin-1-ylsulfonylméthyl)-3-méthylbutanoïque,
le 2-(4-(4-chlorobenzyloxyimino)pipéridin-1-ylsulfonylméthyl)-3-méthylbutanoate de *tert*-butyle,
l'acide 4-(4-(4-chlorophényl)pipérazin-1-yl)sulfonylméthyl)tétrahydropyrane-4-carboxylique,
l'acide 4-(4-pyridyloxypipéridin-1-yl)sulfonylméthyl)tétrahydropyrane-4-carboxylique,
l'acide 4-(4-(4-cyanophénoxy)pipéridin-1-yl)sulfonylméthyltétrahydropyrane-4-carboxylique,
le 4-(*N*-(2-(4-chlorophénoxy)éthyl)-*N*-méthylsulfamoylméthyl)tétrahydropyrane-4-carboxylique,
l'acide 4-(4-(4-chlorophénoxy)pipéridin-1-yl)sulfonylméthyl-tétrahydropyrane-4-carboxylique,
l'acide 2-(*N*-(2-(4-chlorophénoxy)éthyl)-*N*-méthylsulfamoyl-méthyl)-3-méthylbutanoïque,
l'acide 2-(*N*-méthyl-*N*-(2-(4-chlorophénoxy)-2,2-diméthyléthyl)sulfamoylméthyl)-3-méthylbutanoïque,
l'acide 2-(4-(4-cyanophénoxyimino)pipéridin-1-ylsulfonylméthyl)-5-(3,4,4-triméthyl-2,5-dioxoimidazolidin-1-yl)pentanoïque,
l'acide 2-(4-(4-cyanophénoxyimino)pipéridin-1-ylsulfonylméthyl)-3-méthylbutanoïque,
l'acide 2-(4-(4-chlorobenzyloxyimino)pipéridin-1-ylsulfonylméthyl)-3-méthylbutanoïque,
le *N*-hydroxyamide de l'acide 2-(*N*-méthyl-*N*-(2-(4-chlorophénoxy)éthyl)sulfamoylméthyl)-3-méthylbutanoïque,
le *N*-hydroxyamide de l'acide 2-(*N*-méthyl-*N*-(2-(4-chlorophénoxy)-2,2-diméthyléthyl)sulfamoylméthyl)-3-méthylbutanoïque,
le chlorhydrate de *N*-hydroxyamide de l'acide 4-(4-(4-chlorophényl)pipérazin-1-yl)sulfonylméthyl)tétrahydropyrane-4-carboxylique,
le chlorhydrate de *N*-hydroxyamide de l'acide 4-(4-(4-pyridyloxy)pipéridin-1-yl)sulfonylméthyl)tétrahydropyrane-4-carboxylique,
le *N*-hydroxyamide de l'acide 4-(4-(4-cyanophénoxy)pipéridin-1-yl)sulfonylméthyltétrahydropyrane-4-carboxylique,
le *N*-hydroxyamide de l'acide 4-(*N*-(2-(4-chlorophénoxy)éthyl)-*N*-méthylsulfamoylméthyl)-tétrahydropyrane-4-carboxylique,
le *N*-hydroxyamide de l'acide 4-(4-(4-chlorophénoxy)pipéridin-1-yl)sulfonylméthyltétrahydropyrane-4-carboxylique,
le *N*-hydroxyamide de l'acide 2-[4-(4-cyanophénoxyimino)pipéridin-1-ylsulfonylméthyl]-5-(3,4,4-triméthyl-2,5-dioxoimidazolidin-1-yl)pentanoïque,
le *N*-hydroxyamide de l'acide 2-(4-(4-cyanophénoxyimino)pipéridin-1-ylsulfonylméthyl)-3-méthylbutanoïque,
le *N*-hydroxyamide de l'acide 2-(4-(4-chlorobenzyloxyimino)pipéridin-1-ylsulfonylméthyl)-3-méthylbutanoïque,
le *N*-hydroxyamide de l'acide 2-((*N*-éthyl*-N*-(2-méthoxyéthyl)sulfamoylméthyl)-5-(3,4,4-triméthyl-2,5-dioxoimidazolidin-1-yl)pentanoïque,
le *N*-hydroxyamide de l'acide 2-(3,3-diphénylpropylsulfamoylméthyl)-5-(3,4,4-triméthyl-2,5-dioxoimidazolidin-1-yl)pentanoïque,
le *N*-hydroxyamide de l'acide 2-(cyclohexylméthylsulfamoylméthyl)-5-(3,4,4-triméthyl-2,5-dioxoimidazolidin-1-yl)pentanoïque,
le *N*-hydroxyamide de l'acide 2-(1-méthyl-3-phénylpropylsulfamoylméthyl)-5-(3,4,4-triméthyl-2,5-dioxoimidazolidin-1-yl)pentanoïque,
le *N*-hydroxyamide de l'acide 2-((3-phénylpropyl)sulfamoylméthyl)-5-(3,4,4-triméthyl-2,5-dioxoimidazolidin-1-yl)pentanoïque,
le N-hydroxyamide de l'acide 2-((*N*-méthyl-*N*-octylsulfamoyl)méthyl)-5-(3,4,4-triméthyl-2,5-dioxoimidazolidin-1-yl)pentanoïque,
le *N*-hydroxyamide de l'acide 2-(*N*-(2-cyanoéthyl)*-N*-octylsulfamoylméthyl)-5-(3,4,4-triméthyl-2,5-dioxoimidazolidin-1-yl)pentanoïque,
le *N*-hydroxyamide de l'acide 2-(*N*-(2-(4-chlorophénoxy)-1-méthyléthyl)-*N*-méthylsulfamoylméthyl)-5-(3,4,4-triméthyl-2,5-dioxo-imidazolidin-1-yl)pentanoïque,
le *N*-hydroxyamide de l'acide 2-(*N*-méthyl-*N*-phénéthylsulfamoylméthyl)-5-(3,4,4-triméthyl-2,5-dioxoimidazolidin-1-yl)pentanoïque,
le *N*-hydroxyamide de l'acide 2-(*N*-(2-(2-chlorophénoxy)éthyl)-*N*-méthylsulfamoylméthyl) -5-(3,4,4-triméthyl-2,5-dioxoimidazolidin-1-yl)pentanoïque,
le *N*-hydroxyamide de l'acide 2-(*N*-éthyl-*N*-phénylsulfamoylméthyl)-5-(3,4,4-triméthyl-2,5-dioxoimidazolidin-1-yl)pentanoïque, et
le *N*-hydroxyamide de l'acide 2-(*N*-(4-chlorophényl)*-N*-méthylsulfamoylméthyl)-5-(3,4,4-triméthyl-2,5-dioxoimidazolidin-1-yl)pentanoïque.

13. Composé selon l'une quelconque des revendications précédentes, sous forme d'un seul énantiomère ou diastéréo-isomère.

14. Composition pharmaceutique destinée à être utilisée en thérapie, comprenant un composé selon l'une quelconque des revendications précédentes et un diluant ou un support pharmaceutiquement acceptable.

15. Utilisation d'un composé selon l'une quelconque des revendications 1 à 13 pour la fabrication d'un médicament destiné au traitement ou à la prévention d'un état pathologique associé à des métalloprotéinases matricielles ou dont la médiation est assurée par le TNF α ou des enzymes intervenant dans la diffusion de la L-sélectine, du CD23, des récepteurs de TNF, des récepteurs de IL-6 ou des récepteurs de IL-1.

16. Utilisation selon la revendication 15, dans laquelle l'état pathologique est choisi parmi un cancer, une inflammation et des maladies inflammatoires, une dégénération tissulaire, une maladie périodontale, une maladie ophtalmologique, des troubles dermatologiques, la fièvre, des effets cardiovasculaires, une hémorragie, une coagulation et une réponse de phase aiguë, la cachexie, l'anorexie, une infection aiguë, une infection VIH, des états de choc, des réactions de greffe contre hôte, une maladie auto-immune, une lésion de reperfusion, la méningite, la migraine et une anti-thrombose indépendante de l'aspirine.

17. Utilisation selon la revendication 15, dans laquelle l'état pathologique est choisi parmi une croissance tumorale, l'angiogénèse, une invasion et une extension tumorales, des métastases, une ascite maligne et un épanchement pleural malin.

18. Utilisation selon la revendication 15, dans laquelle l'état pathologique est choisi parmi une ischémie cérébrale, une maladie cardiaque ischémique, la polyarthrite rhumatoïde, l'arthrose, l'ostéoporose, l'asthme, la sclérose en plaques, la neurodégénération, la maladie d'Alzheimer, l'athérosclérose, un ictus, une vasculite, la maladie de Crohn et la rectocolite hémorragique.

19. Utilisation selon la revendication 15, dans laquelle l'état pathologique est choisi parmi l'ulcération cornéenne, la rétinopathie et la cicatrisation d'une plaie chirurgicale.

20. Utilisation selon la revendication 15, dans laquelle l'état pathologique est choisi parmi le psoriasis, la dermatite atopique, des ulcères chroniques et l'épidermolyse bulleuse.

21. Utilisation selon la revendication 15, dans laquelle l'état pathologique est choisi parmi la périodontite et la gingivite.

22. Utilisation selon la revendication 15, dans laquelle l'état pathologique est choisi parmi la rhinite, la conjonctivite allergique, l'eczéma et l'anaphylaxie.

23. Utilisation selon la revendication 15, dans laquelle l'état pathologique est choisi parmi la resténose, la défaillance cardiaque congestive, l'endométriose, l'athérosclérose et l'endosclérose.

24. Utilisation selon la revendication 15, dans laquelle l'état pathologique est choisi parmi la pelvipéritonite, la dégénération maculaire liée à l'âge et la résorption osseuse induite par un cancer.

25. Utilisation selon la revendication 15, dans laquelle l'état pathologique est une maladie pulmonaire.

26. Utilisation selon la revendication 25, dans laquelle l'état pathologique est choisi parmi la fibrose kystique, le syndrome de détresse respiratoire de l'adulte (SDRA), l'emphysème, la bronchiolite oblitérante avec pneumonie organisée (BOOP), la fibrose pulmonaire idiopathique (FPI), un dommage alvéolaire diffus, la granulomatose pulmonaire à cellules de Langerhan, la lymphangioléiomyomatose pulmonaire (LAM) et la maladie pulmonaire obstructive chronique (MPOC).
